# EUROPEAN PATENT APPLICATION

(11) **EP 4 417 595 A1**
(43) Date of publication of application: **21.08.2024**
(21) Application number: 22880726.9
(22) Date of filing: 20.09.2022
(51) Int. Cl.: C07C 45/67, C07C 49/403, C07B 61/00

(54) **COMPOUND PRODUCING METHOD INCLUDING STEP FOR ISOMERIZING ALPHA, BETA-UNSATURATED KETONE**

(30) Priority: 12.10.2021 JP 2021167116
(71) Applicant: Kao Corporation, Tokyo 103-8210 (JP)
(72) Inventor: MORITA, Kengo, Wakayama-shi, Wakayama 640-8580 (JP)
(74) Representative: Hoffmann Eitle
(86) International application number: PCT/JP2022/034986
(87) International publication number: WO 2023/063030

(57) **Abstract**

Provided is a method for isomerizing an α,β-unsaturated ketone, capable of shortening the reaction time and improving the reaction yield. The present invention provides a method for producing a compound represented by General Formula (I) below, including a step of isomerizing a compound represented by General Formula (II) below in the presence of molecular hydrogen and/or a hydrogen source, a platinum group metal catalyst, and a simple substance of a Group 16 element (excluding oxygen) or a compound thereof.

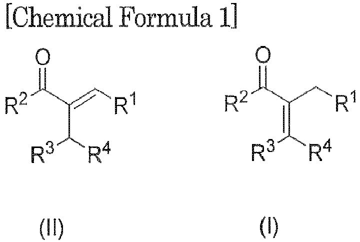

## Description

### Technical Field

The present invention relates to a compound producing method including a step of isomerizing an α,β-unsaturated ketone.

### Background Art

Alkyl(3-oxo-2-alkylcycloalkyl) acetates, especially methyl dihydrojasmonates (MDJ), are useful fragrance materials with floral, jasmine-like aromas.

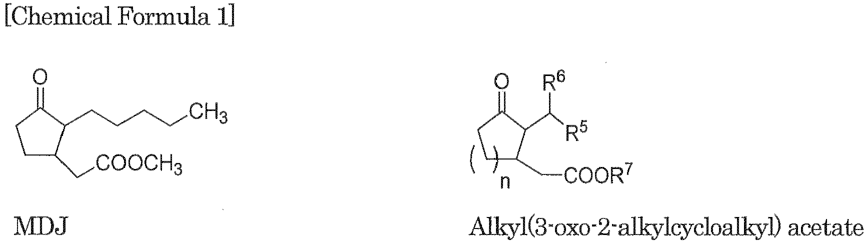

For example, it has already been reported that MDJ is obtained by an aldol reaction and dehydration and isomerization reactions of cyclopentanone (1) and pentanal (2) to obtain a compound (3) and then a reaction of the compound with a diethyl malonate (JP 2009-269910A).

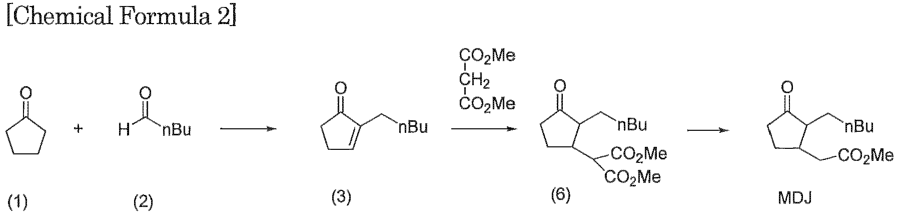

The aldol reaction and the dehydration and isomerization reactions specifically proceed as follows.

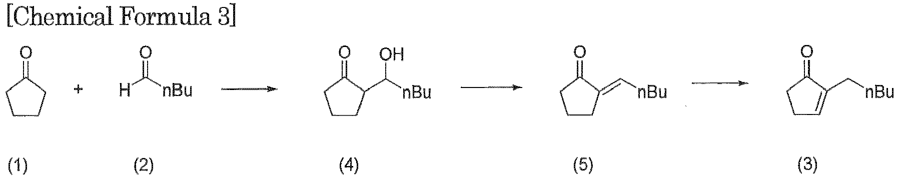

The aldol reaction of cyclopentanone (1) and pentanal (2) yields keto alcohol (4). Dehydration of the keto alcohol (4) yields an α,β-unsaturated ketone compound (5). Isomerization of the α,β-unsaturated ketone compound (5) yields an α,β-unsaturated ketone compound (3).

For example, JP 2009-269910A discloses a method for producing 2-alkyl-2-cycloalken-1-one by dehydrating and isomerizing 2-(1-hydroxyalkyl)-cycloalkan-1-one in the coexistence of an acid and a platinum group metal catalyst.

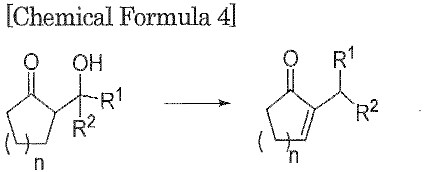

In addition, JP 2016-509991A discloses a production method including isomerization in the presence of a catalyst system containing: i) a metal selected from palladium (Pd) and platinum (Pt); and ii) molecular hydrogen or a hydrogen source.

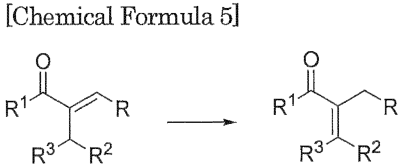

Furthermore, JP H7-69938A discloses a method for isomerizing olefin by using a platinum group catalyst treated with an organic sulfur compound prior to charge into a reactor in an isomerization reaction of a double bond using a platinum group metal catalyst and hydrogen.

### Summary of the Invention

The present invention provides a method for producing a compound represented by General Formula (I) below, including a step of isomerizing a compound represented by General Formula (II) below in the presence of molecular hydrogen and/or a hydrogen source, a platinum group metal catalyst, and a simple substance of a Group 16 element (excluding oxygen) or a compound thereof.

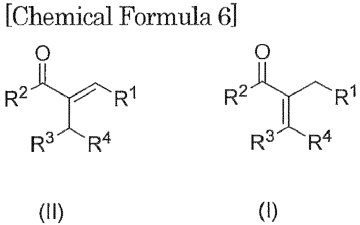

In the formulas, R¹, R², and R⁴ simultaneously or independently represent a hydrogen atom, a linear or branched alkyl group having 1 to 8 carbon atoms, or a linear or branched alkenyl group having 2 to 8 carbon atoms, and
R³ represents a linear or branched alkyl group having 1 to 8 carbon atoms or a linear or branched alkenyl group having 2 to 8 carbon atoms, or
R¹ and R⁴ simultaneously or independently represent a hydrogen atom, a linear or branched alkyl group having 1 to 8 carbon atoms, or a linear or branched alkenyl group having 2 to 8 carbon atoms, and
R² and R³, taken together, represent an alkanediyl group having 2 to 9 carbon atoms or represent an alkanediyl group having 2 to 9 carbon atoms substituted with one or two alkyl groups having 1 to 5 carbon atoms.

### Description of the Invention

However, the methods of JP 2009-269910A and JP 2016-509991A are problematic in that the selectivity of the isomerization reaction is reduced because the presence of molecular hydrogen or a hydrogen source in isomerization of a double bond results in byproduction of a compound in which the double bond moiety is hydrogenated. Furthermore, if the amount of hydrogen is reduced to suppress hydrogenation, the reactivity of the isomerization reaction decreases. Increasing the amount of platinum group catalyst can achieve both reactivity and selectivity of the isomerization reaction, but the production cost increases.

Furthermore, the method of JP H7-69938Ais intended for olefins and differs from that for isomerization of compounds obtained by dehydrating keto alcohols. In addition, this method has the problem of low yield.

Accordingly, it is an object of the present invention to provide a method for isomerizing an α,β-unsaturated ketone, capable of shortening the reaction time and improving the reaction yield.

The inventors of the present invention surprisingly found that the presence of sulfur, which is a Group 16 element (excluding oxygen), during isomerization of an α,β-unsaturated ketone using a platinum group catalyst shortens the reaction time and improves the reaction yield. The inventors have accomplished the method of the present invention based on this finding.

That is to say, the present invention is directed to a method for producing a compound represented by General Formula (I) below, including a step of isomerizing a compound represented by General Formula (II) below in the presence of molecular hydrogen and/or a hydrogen source, a platinum group metal catalyst, and a simple substance of a Group 16 element (excluding oxygen) or a compound thereof.

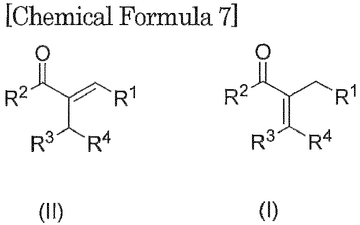

In the formulas, R¹, R², and R⁴ simultaneously or independently represent a hydrogen atom, a linear or branched alkyl group having 1 to 8 carbon atoms, or a linear or branched alkenyl group having 2 to 8 carbon atoms, and
R³ represents a linear or branched alkyl group having 1 to 8 carbon atoms or a linear or branched alkenyl group having 2 to 8 carbon atoms, or
R¹ and R⁴ simultaneously or independently represent a hydrogen atom, a linear or branched alkyl group having 1 to 8 carbon atoms, or a linear or branched alkenyl group having 2 to 8 carbon atoms, and
R² and R³, taken together, represent an alkanediyl group having 2 to 9 carbon atoms or represent an alkanediyl group having 2 to 9 carbon atoms substituted with one or two alkyl groups having 1 to 5 carbon atoms.

The method of the present invention can shorten the reaction time in a method for isomerizing an α,β-unsaturated ketone and improve the reaction yield in the method. Moreover, the amount of platinum metal catalyst can be reduced.

As described above, the present invention is directed to a method for producing a compound represented by General Formula (I) below, including a step of isomerizing a compound represented by General Formula (II) below in the presence of molecular hydrogen and/or a hydrogen source, a platinum group metal catalyst, and a simple substance of a Group 16 element (excluding oxygen) or a compound thereof.

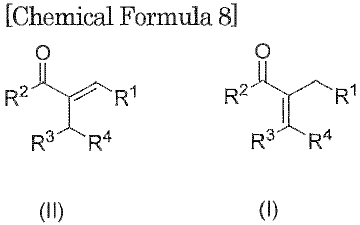

In the formulas, R¹, R², and R⁴ simultaneously or independently represent a hydrogen atom, a linear or branched alkyl group having 1 to 8 carbon atoms, or a linear or branched alkenyl group having 2 to 8 carbon atoms, and R³ represents a linear or branched alkyl group having 1 to 8 carbon atoms or a linear or branched alkenyl group having 2 to 8 carbon atoms, or
R¹ and R⁴ simultaneously or independently represent a hydrogen atom, a linear or branched alkyl group having 1 to 8 carbon atoms, or a linear or branched alkenyl group having 2 to 8 carbon atoms, and
R² and R³, taken together, represent an alkanediyl group having 2 to 9 carbon atoms or represent an alkanediyl group having 2 to 9 carbon atoms substituted with one or two alkyl groups having 1 to 5 carbon atoms.

Furthermore, the present invention is directed to the method for producing a compound represented by General Formula (1), further including a step of dehydrating the compound represented by General Formula (III) above in the presence of an acid, thereby obtaining the compound represented by General Formula (II) above.

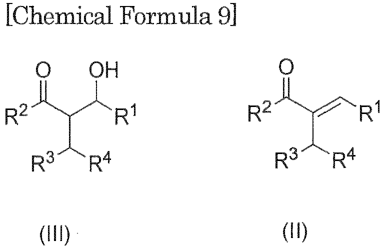

In the formulas, R¹, R², and R⁴ simultaneously or independently represent a hydrogen atom, a linear or branched alkyl group having 1 to 8 carbon atoms, or a linear or branched alkenyl group having 2 to 8 carbon atoms, and
R³ represents a linear or branched alkyl group having 1 to 8 carbon atoms or a linear or branched alkenyl group having 2 to 8 carbon atoms, or
R¹ and R⁴ simultaneously or independently represent a hydrogen atom, a linear or branched alkyl group having 1 to 8 carbon atoms, or a linear or branched alkenyl group having 2 to 8 carbon atoms, and
R² and R³, taken together, represent an alkanediyl group having 2 to 9 carbon atoms or represent an alkanediyl group having 2 to 9 carbon atoms substituted with one or two alkyl groups having 1 to 5 carbon atoms.

Furthermore, the present invention is directed to a method for producing a compound of Formula (VII), including Steps 1 and 2 below.

Step 1: Step of dehydrating and isomerizing a compound represented by General Formula (IV) below in the presence of molecular hydrogen and/or a hydrogen source, an acid, a platinum group metal catalyst, and a simple substance of a Group 16 element (excluding oxygen) or a compound thereof, thereby obtaining a compound represented by General Formula (V) below

Step 2: Step of reacting the compound represented by General Formula (V) obtained in Step 1 with a malonic acid diester represented by General Formula (VI) below and then with water, thereby obtaining a compound represented by General Formula (VII) below

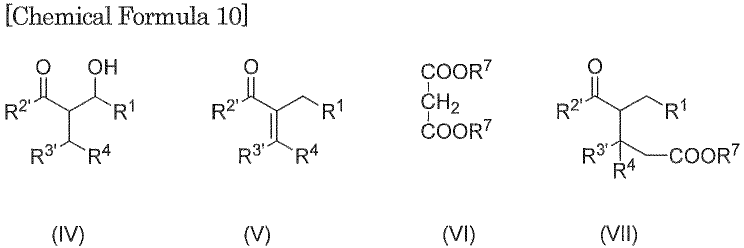

In the formulas, R¹ and R⁴ simultaneously or independently represent a hydrogen atom, a linear or branched alkyl group having 1 to 8 carbon atoms, or a linear or branched alkenyl group having 2 to 8 carbon atoms,

R²' and R³', taken together, represent an alkanediyl group having 2 to 9 carbon atoms or represent an alkanediyl group having 2 to 9 carbon atoms substituted with one or two alkyl groups having 1 to 5 carbon atoms, and

R⁷ represents an alkyl group having 1 to 3 carbon atoms, and two R⁷s are optionally the same or different.

In this specification, the "linear or branched alkyl group having 1 to 8 carbon atoms" may refer to, for example, methyl, ethyl, n-propyl, isopropyl, 2-methylpropyl, n-butyl, i-butyl, sec-butyl, t-butyl, n-pentyl, an i-pentyl group, a sec-pentyl group, a t-pentyl group, a 2-methylbutyl group, n-hexyl, a 1-methylpentyl group, a 2-methylpentyl group, a 3-methylpentyl group, a 4-methylpentyl group, a 1-ethylbutyl group, a 2-ethylbutyl group, a 3-ethylbutyl group, a 1,1-dimethylbutyl group, a 2,2-dimethylbutyl group, a 3,3-dimethylbutyl group, a 1-ethyl-1-methylpropyl group, n-heptyl, a 1-methylhexyl group, a 2-methylhexyl group, a 3-methylhexyl group, a 4-methylhexyl group, a 5-methylhexyl group, a 1-ethylpentyl group, a 2-ethylpentyl group, a 3-ethylpentyl group, a 4-ethylpentyl group, a 1,1-dimethylpentyl group, a 2,2-dimethylpentyl group, a 3,3-dimethylpentyl group, a 4,4-dimethylpentyl group, a 1-propylbutyl group, n-octyl, or the like. The "linear alkyl group having 1 to 8 carbon atoms" may refer to, for example, methyl, ethyl, n-propyl, n-butyl, n-pentyl, n-hexyl, n-heptyl, n-octyl, or the like. The "branched alkyl group having 1 to 8 carbon atoms" may refer to, for example, isopropyl, i-butyl, sec-butyl, an i-pentyl group, a sec-pentyl group, a t-pentyl group, 2-methylpropyl, a 2-methylbutyl group, t-butyl, a 1-methylpentyl group, a 2-methylpentyl group, a 3-methylpentyl group, a 4-methylpentyl group, a 1-ethylbutyl group, a 2-ethylbutyl group, a 3-ethylbutyl group, a 1,1-dimethylbutyl group, a 2,2-dimethylbutyl group, a 3,3-dimethylbutyl group, a 1-ethyl-1-methylpropyl group, a 1-methylhexyl group, a 2-methylhexyl group, a 3-methylhexyl group, a 4-methylhexyl group, a 5-methylhexyl group, a 1-ethylpentyl group, a 2-ethylpentyl group, a 3-ethylpentyl group, a 4-ethylpentyl group, a 1,1-dimethylpentyl group, a 2,2-dimethylpentyl group, a 3,3-dimethylpentyl group, a 4,4-dimethylpentyl group, a 1-propylbutyl group, or the like. The "alkyl group having 1 to 8 carbon atoms" of the "linear or branched alkyl group having 1 to 8 carbon atoms" is preferably an alkyl group having 1 to 5 carbon atoms, and more preferably an alkyl group having 1 to 3 carbon atoms.

In this specification, the "alkyl group having 1 to 5 carbon atoms" may refer to, for example, methyl, ethyl, n-propyl, isopropyl, n-butyl, t-butyl, pentyl, or the like. The "alkyl group having 1 to 5 carbon atoms" is preferably an alkyl group having 1 to 3 carbon atoms, and more preferably an alkyl group having 1 or 2 carbon atoms.

In this specification, the "alkyl group having 1 to 3 carbon atoms" may refer to, for example, methyl, ethyl, n-propyl, isopropyl, or the like. The "alkyl group having 1 to 3 carbon atoms" is preferably an alkyl group having 1 or 2 carbon atoms.

In this specification, the "linear or branched alkenyl group having 2 to 8 carbon atoms" may refer to, for example, vinyl, allyl, isopropenyl, 1-butenyl, 2-butenyl, 3-butenyl, 1-pentenyl, 2-pentenyl, 3-pentenyl, 4-pentenyl, 1-hexenyl, 2-hexenyl, 3-hexenyl, 4-hexenyl, 5-hexenyl, or the like. The "alkenyl group having 2 to 8 carbon atoms" of the "linear or branched alkenyl group having 2 to 8 carbon atoms" is preferably an alkenyl group having 2 to 6 carbon atoms, and more preferably an alkenyl group having 2 to 4 carbon atoms.

In this specification, the "alkanediyl group having 2 to 9 carbon atoms" may refer to, for example, a linear alkylene group having 2 to 9 carbon atoms such as ethylene, a propane-1,3-diyl group, a butane-1,4-diyl group, a group, a hexane-1,6-diyl group, a heptane-1,7-diyl group, an octane-1,8-diyl group, or a nonane-1,9-diyl group. The "alkanediyl group having 2 to 9 carbon atoms" is preferably an alkanediyl group having 2 to 7 carbon atoms, and more preferably an alkanediyl group having 2 to 5 carbon atoms.

In this specification, the "alkanediyl group having 2 to 9 carbon atoms substituted with one or two alkyl groups having 1 to 5 carbon atoms" may refer to, for example, an ethane-1,1-diyl group, a propane-1,1-diyl group, a propane-1,2-diyl group, a propane-2,2-diyl group, a pentane-2,4-diyl group, a 2-methylpropane-1,3-diyl group, a 2-methylpropane-1,2-diyl group, a pentane-1,4-diyl group, or 2-methyl butane-1,4-diyl. The "alkanediyl group having 2 to 9 carbon atoms substituted with one or two alkyl groups having 1 to 5 carbon atoms" is preferably an alkanediyl group having 2 to 7 carbon atoms substituted with one or two alkyl groups having 1 to 5 carbon atoms, more preferably an alkanediyl group having 2 to 7 carbon atoms substituted with one or two alkyl groups having 1 to 3 carbon atoms, and even more preferably an alkanediyl group having 2 to 5 carbon atoms substituted with one or two alkyl groups having 1 to 3 carbon atoms.

### <Compound Represented by General Formula (II) and Compound Represented by General Formula (I)>

In the method for producing the compound represented by General Formula (I) of the present invention (also referred to as a "compound of Formula (I)" or a "compound (I)" hereinafter), a compound represented by General Formula (II) (also referred to as a "compound of Formula (II)" or a "compound (II)" hereinafter) is used as a raw material.

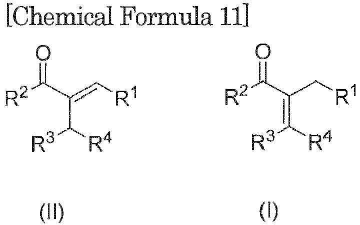

In Formulas (I) and (II) above,
R¹, R², and R⁴ simultaneously or independently represent a hydrogen atom, a linear or branched alkyl group having 1 to 8 carbon atoms, or a linear or branched alkenyl group having 2 to 8 carbon atoms, and
R³ represents a linear or branched alkyl group having 1 to 8 carbon atoms or a linear or branched alkenyl group having 2 to 8 carbon atoms, or
R¹ and R⁴ simultaneously or independently represent a hydrogen atom, a linear or branched alkyl group having 1 to 8 carbon atoms, or a linear or branched alkenyl group having 2 to 8 carbon atoms, and
R² and R³, taken together, represent an alkanediyl group having 2 to 9 carbon atoms or represent an alkanediyl group having 2 to 9 carbon atoms substituted with one or two alkyl groups having 1 to 5 carbon atoms.

In Formulas (I) and (II) above, it is preferable that:
R¹, R², and R⁴ simultaneously or independently represent a hydrogen atom, a linear or branched alkyl group having 1 to 6 carbon atoms, or a linear or branched alkenyl group having 2 to 6 carbon atoms, and
R³ represents a linear or branched alkyl group having 1 to 6 carbon atoms, or a linear or branched alkenyl group having 2 to 6 carbon atoms, or
R¹ and R⁴ simultaneously or independently represent a hydrogen atom, a linear or branched alkyl group having 1 to 6 carbon atoms, or a linear or branched alkenyl group having 2 to 6 carbon atoms, and
R² and R³, taken together, represent an alkanediyl group having 2 to 7 carbon atoms or represent an alkanediyl group having 2 to 7 carbon atoms substituted with one or two alkyl groups having 1 to 5 carbon atoms.

In Formulas (I) and (II) above,
R¹ is preferably an alkyl group having 1 to 4 carbon atoms, and more preferably an n-butyl group.

In Formulas (I) and (II) above,
R⁴ is preferably an alkyl group having 1 to 4 carbon atoms, and more preferably a hydrogen atom.

In Formulas (I) and (II) above,
R² and R³, taken together, are preferably an alkanediyl group having 2 to 9 carbon atoms, more preferably an alkanediyl group having 2 to 4 carbon atoms, and even more preferably an alkanediyl group having 2 carbon atoms.

In Formulas (I) and (II) above, it is more preferable that R¹ is an n-butyl group, R² and R³, taken together, represent an alkanediyl group having 2 carbon atoms, and R⁴ is a hydrogen atom.

The compound (I) may specifically refer to 2-propyl-2-cyclopenten-1-one, 2-butyl-2-cyclopenten-1-one, 2-pentyl-2-cyclopenten-1-one, 2-hexyl-2-cyclopenten-1-one, 2-(1-methylbutyl)-2-cyclopenten -I-one, 2-(2-methylbutyl)-2-cyclopenten-1-one, 2-cyclopentyl-2-cyclopenten-1-one, 2-cyclohexyl-2-cyclopenten-1-one, 2-propyl-2-cyclohexen-1-one, 2-butyl-2-cyclohexen-1-one, 2-pentyl-2-cyclohexen-1-one, 2-hexyl-2-cyclohexen-1-one, 2-(1-methylbutyl)-2-cyclohexen-1-one, 2-(2-methylbutyl)-2-cyclohexen-1-one, 2-cyclopentyl-2-cyclohexen-1-one, 2-cyclohexyl-2-cyclohexen-1-one, or the like. Among these, 2-propyl-2-cyclopenten-1-one, 2-butyl-2-cyclopenten-1-one, 2-pentyl-2-cyclopenten-1-one, and 2-hexyl-2-cyclopenten-1-one are preferred, and 2-pentyl-2-cyclopenten-1-one is more preferred.

The compound (II) may specifically refer to 2-propylidenecyclopentan-1-one, 2-butylidenecyclopentan-1-one, 2-pentylidenecyclopentan-1-one, 2-hexylidenecyclopentan-1-one, 2-cyclopentylidenecyclopentan-1-one, 2-cyclohexylidenecyclopentan-1-one, 2-propylidenecyclohexan-1-one, 2-butylidenecyclohexan-1-one, 2-pentylidenecyclohexan-1-one, 2-hexylidenecyclohexan-1-one, 2-cyclopentylidenecyclohexan-1-one, 2-cyclohexylidenecyclohexan-1-one, or the like. Among these, 2-propylidenecyclopentan-1-one, 2-butylidenecyclopentan-1-one, 2-pentylidenecyclopentan-1-one, and 2-hexylidenecyclopentan-1-one are preferred, and 2-pentylidenecyclopentan-1-one is more preferred.

### <Compound Represented by General Formula (III)>

In the method for producing the compound represented by General Formula (I) of the present invention (also referred to as a "compound of Formula (I)" or a "compound (I)" hereinafter), the compound represented by General Formula (III) (also referred to as a "compound of Formula (III)" or a "compound (III)" hereinafter) is used as a raw material.

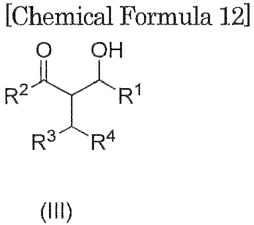

In Formula (III) above,
R¹, R², and R⁴ simultaneously or independently represent a hydrogen atom, a linear or branched alkyl group having 1 to 8 carbon atoms, or a linear or branched alkenyl group having 2 to 8 carbon atoms, and
R³ represents a linear or branched alkyl group having 1 to 8 carbon atoms or a linear or branched alkenyl group having 2 to 8 carbon atoms, or
R¹ and R⁴ simultaneously or independently represent a hydrogen atom, a linear or branched alkyl group having 1 to 8 carbon atoms, or a linear or branched alkenyl group having 2 to 8 carbon atoms, and
R² and R³, taken together, represent an alkanediyl group having 2 to 9 carbon atoms or represent an alkanediyl group having 2 to 9 carbon atoms substituted with one or two alkyl groups having 1 to 5 carbon atoms.

In Formula (III) above, it is preferable that:
R¹, R², and R⁴ simultaneously or independently represent a hydrogen atom, a linear or branched alkyl group having 1 to 6 carbon atoms, or a linear or branched alkenyl group having 2 to 6 carbon atoms, and
R³ represents a linear or branched alkyl group having 1 to 8 carbon atoms or a linear or branched alkenyl group having 2 to 8 carbon atoms, or
R¹ and R⁴ simultaneously or independently represent a hydrogen atom, a linear or branched alkyl group having 1 to 6 carbon atoms, or a linear or branched alkenyl group having 2 to 6 carbon atoms, and
R² and R³, taken together, represent an alkanediyl group having 2 to 7 carbon atoms or represent an alkanediyl group having 2 to 7 carbon atoms substituted with one or two alkyl groups having 1 to 5 carbon atoms.

In Formula (III) above, it is more preferable that:
R¹ and R⁴ simultaneously or independently represent a hydrogen atom, a linear or branched alkyl group having 1 to 8 carbon atoms, or a linear or branched alkenyl group having 2 to 8 carbon atoms, and
R² and R³, taken together, represent an alkanediyl group having 2 to 9 carbon atoms.

In Formula (III) above, R¹ is preferably an alkyl group having 1 to 4 carbon atoms, and more preferably an n-butyl group.

In Formula (III) above, R⁴ is preferably an alkyl group having 1 to 4 carbon atoms, and more preferably a hydrogen atom.

In Formula (III) above, R² and R³, taken together, are preferably an alkanediyl group having 2 to 9 carbon atoms, more preferably an alkanediyl group having 2 to 4 carbon atoms, and even more preferably an alkanediyl group having 2 carbon atoms.

In Formula (III) above, it is more preferable that R¹ is an n-butyl group, both R² and R³ represent an alkanediyl group having 2 carbon atoms, and R⁴ is a hydrogen atom.

The compound (III) may specifically refer to 2-(1-hydroxypropyl)-cyclopentanone, 2-(1-hydroxybutyl)-cyclopentanone, 2-(1-hydroxypentyl)-cyclopentan-1-one, 2-(1-hydroxyhexyl)-cyclopentanone, 2-(1-hydroxy-1-methylbutyl)-cyclopentanone, 2-(1-hydroxy-2-methylbutyl)-cyclopentanone, 2-(1-hydroxycyclopentyl)-cyclopentanone, 2-(1-hydroxycyclohexyl)-cyclopentanone, 2-(1-hydroxypropyl)-cyclohexanone, 2-(1-hydroxybutyl)-cyclohexanone, 2-(1-hydroxypentyl)-cyclohexanone, 2-(1-hydroxyhexyl)-cyclohexanone, 2-(1-hydroxy-1-methylbutyl)-cyclohexanone, 2-(1-hydroxy-2-methylbutyl)-cyclohexanone, 2-(1-hydroxycyclopentyl)-cyclohexanone, 2-(1-hydroxycyclohexyl)-cyclohexanone, or the like. Among these, 2-(1-hydroxypropyl)-cyclopentanone, 2-(1-hydroxybutyl)-cyclopentanone, 2-(1-hydroxypentyl)-cyclopentan-1-one, and 2-(1-hydroxyhexyl)-cyclopentanone are preferred, and 2-(1-hydroxypentyl)-cyclopentan-1-one is more preferred.

### <Compound Represented by General Formula (IV), Compound Represented by General Formula (V), Compound Represented by General Formula (VI), and Compound Represented by General Formula (VII)>

In the method for producing the compound represented by General Formula (VII) of the present invention (also referred to as a "compound of Formula (VII)" or a "compound (VII)" hereinafter), the compound represented by General Formula (IV) (also referred to as a "compound of Formula (IV)" or a "compound (IV)" hereinafter), the compound represented by General Formula (V) (also referred to as a "compound of Formula (V)" or a "compound (V)" hereinafter), and the compound represented by General Formula (VI) (also referred to as a "compound of Formula (VI)" or a "compound (VI)" hereinafter) are used as a raw material.

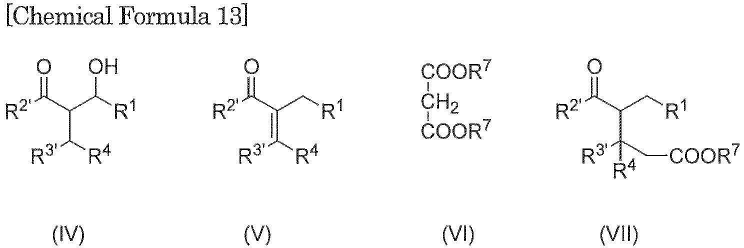

In Formulas (IV), (V), (VI), and (VII) above,
R¹ and R⁴ simultaneously or independently represent a hydrogen atom, a linear or branched allcyl group having 1 to 8 carbon atoms, or a linear or branched alkenyl group having 2 to 8 carbon atoms,
R²' and R³', taken together, represent an alkanediyl group having 2 to 9 carbon atoms or represent an alkanediyl group having 2 to 9 carbon atoms substituted with one or two alkyl groups having 1 to 5 carbon atoms, and
R⁷ represents an alkyl group having 1 to 3 carbon atoms, and two R⁷s can be the same or different.

In Formulas (IV), (V), (VI), and (VII) above, it is preferable that:
R¹ and R⁴ simultaneously or independently represent a hydrogen atom, a linear or branched alkyl group having 1 to 6 carbon atoms, or a linear or branched alkenyl group having 2 to 6 carbon atoms,
R²' and R³', taken together, represent an alkanediyl group having 2 to 7 carbon atoms or represent an alkanediyl group having 2 to 7 carbon atoms substituted with one or two alkyl groups having 1 to 5 carbon atoms, and
R⁷ represents an alkyl group having 1 to 3 carbon atoms, and two R⁷s can be the same or different.

In Formulas (IV), (V), (VI), and (VII) above, R¹ is preferably an alkyl group having 1 to 4 carbon atoms, and more preferably an n-butyl group.

In Formulas (IV), (V), (VI), and (VII) above, R⁴ is preferably an alkyl group having 1 to 4 carbon atoms, and more preferably a hydrogen atom.

In Formulas (IV), (V), (VI), and (VII) above, R²' and R³', taken together, are preferably an alkanediyl group having 2 to 9 carbon atoms, more preferably an alkanediyl group having 2 to 4 carbon atoms, and even more preferably an alkanediyl group having 2 carbon atoms.

In Formulas (IV), (V), (VI), and (VII) above, R⁷ is preferably a methyl group.

In Formulas (IV), (V), (VI), and (VII) above, it is preferable that R¹ is an n-butyl group, R²' and R³', taken together, represent an alkanediyl group having 2 carbon atoms, R⁴ is a hydrogen atom, and R⁷ is a methyl group.

The compound (IV) may specifically refer to 2-(1-hydroxypropyl)-cyclopentanone, 2-(1-hydroxybutyl)-cyclopentanone, 2-(1-hydroxypentyl)-cyclopentan-1-one, 2-(1-hydroxyhexyl)-cyclopentanone, 2-(1-hydroxy-1-methylbutyl)-cyclopentanone, 2-(1-hydroxy-2-methylbutyl)-cyclopentanone, 2-(1-hydroxycyclopentyl)-cyclopentanone, 2-(1-hydroxycyclohexyl)-cyclopentanone, 2-(1-hydroxypropyl)-cyclohexanone, 2-(1-hydroxybutyl)-cyclohexanone, 2-(1-hydroxypentyl)-cyclohexanone, 2-(1-hydroxyhexyl)-cyclohexanone, 2-(1- hydroxy-1-methylbutyl)-cyclohexanone, 2-(1-hydroxy-2-methylbutyl)-cyclohexanone, 2-(1-hydroxycyclopentyl)-cyclohexanone, 2-(1-hydroxycyclohexyl)-cyclohexanone, or the like. Among these, 2-(1-hydroxypropyl)-cyclopentanone, 2-(1-hydroxybutyl)-cyclopentanone, 2-(1-hydroxypentyl)-cyclopentan-1-one, and 2-(1-hydroxyhexyl)-cyclopentanone are preferred, and 2-(1-hydroxypentyl)-cyclopentan-1-one is more preferred.

The compound (V) may specifically refer to 2-propyl-2-cyclopenten-1-one, 2-butyl-2-cyclopenten-1-one, 2-pentyl-2-cyclopenten-1-one, 2-hexyl-2-cyclopenten-1-one, 2-(1-methylbutyl)-2-cyclopenten-1-one, 2-(2-methylbutyl)-2-cyclopenten-1-one, 2-cyclopentyl-2-cyclopenten-1-one, 2-cyclohexyl-2-cyclopenten- 1-one, 2-propyl-2-cyclohexen-1-one, 2-butyl-2-cyclohexen-1-one, 2-pentyl-2-cyclohexen-1-one, 2-hexyl-2-cyclohexen-1-one, 2-(1-methylbutyl)-2-cyclohexen-1-one, 2-(2-methylbutyl)-2-cyclohexen-1-one, 2-cyclopentyl-2-cyclohexen-1-one, 2-cyclohexyl-2-cyclohexen-1-one, or the like. Among these, 2-propyl-2-cyclopenten-1-one, 2-butyl-2-cyclopenten-1-one, 2-pentyl-2-cyclopenten-1-one, and 2-hexyl-2-cyclopenten-1-one are preferred, and 2-pentyl-2-cyclopenten-1-one is more preferred.

The compound (VI) may specifically refer to dimethyl malonate, diethyl malonate, dibutyl malonate, or the like. Among these, diethyl malonate is preferred.

The compound (VII) may specifically refer to methyl dihydrojasmonate or the like.

### [Method for Producing Compound of Formula (I)]

In the present invention, the compound of Formula (I) can be produced through a step of isomerizing the compound of Formula (II) in the presence of molecular hydrogen and/or a hydrogen source, a platinum group metal catalyst, and a simple substance of a Group 16 element (excluding oxygen) or a compound thereof.

### <Molecular Hydrogen and/or Hydrogen Source>

The hydrogen source can be a hydrogen transfer agent. The hydrogen transfer agent is, for example, tetralin, formic acid, limonene, or cyclohexanol. The molecular hydrogen and the hydrogen source can be used together.

The molecular hydrogen can be used in a state of being mixed with inert gas. The inert gas can be selected from the group consisting of, for example, nitrogen, argon, and helium. If the molecular hydrogen and the inert gas are used in a mixed state, the mixing ratio thereof (volume ratio, molecular hydrogen / inert gas) is preferably 1/10 or more, more preferably 1/5 or more, and even more preferably 1/3 or more, from the viewpoint of reactivity, is preferably 10/1 or less, more preferably 5/1 or less, and even more preferably 3/1 or less, from the viewpoint of suppressing side reactions, and is preferably 10/1 to 1/10, more preferably 5/1 to 1/5, and even more preferably 3/1 to 1/3.

### <Platinum Group Metal Catalyst>

The platinum group metal catalyst for use in the present invention is a catalyst containing, as a main component, one or more metal components selected from the group consisting of osmium (Os), ruthenium (Ru), iridium (Ir), rhodium (Rh), platinum (Pt), and palladium (Pd), which are included in the elements of Periods 5 and 6 in Groups 8 to 10 of the Periodic Table. Among these metal components, Pt and Pd are preferred, and Pd is more preferred, from the viewpoint of catalytic activity and the like. These metal components can be used alone or in a combination of two or more. In this case, "containing as a main component" means that the component is contained in an amount of preferably 50 mol% or more, more preferably 70 mol% or more, even more preferably 90 mol% or more, and even more preferably 95 mol% or more, in the catalyst metal component.

These platinum group metal catalysts may contain other metal components or secondary amounts of auxiliary catalysts. Examples of the other metal components include elements of Period 4 in Groups 4 to 11 of the Periodic Table, such as Ti, V, Cr, Mn, Fe, Co, Ni, and Cu, and W, Ag, and Au.

The catalyst can be prepared and used as appropriate in supported, Raney, soluble, powdered, granular, or other forms.

Supported catalysts are catalysts in which a metal component is supported on a support to improve physical properties such as catalyst durability. Supported catalysts can be prepared using known methods such as precipitation, ion exchange, evaporation-drying, spray-drying, and kneading. Examples of the support include carbon (activated carbon), alumina, silica, silica-alumina, barium sulfate, and calcium carbonate. Among these, carbon (activated carbon), silica, alumina, and silica-alumina are preferred.

If a palladium catalyst is used as a catalyst, specific examples thereof include palladium on carbon, palladium-supported alumina, palladium-supported barium sulfate, and palladium-supported calcium carbonate. Among these, palladium on carbon and palladium-supported alumina are preferred because of their high reactivity and ease of recovering the palladium catalyst after the reaction, and palladium on carbon is more preferred from the viewpoint of availability, ease of handling, reactivity, and the like.

It is preferable that the amount of metal component supported in a supported catalyst is typically about 0.1 to 70 mass% based on the total amount of the support and the supported metal component, from the viewpoint of catalytic activity.

The amount of platinum group metal catalyst used can be optimized as appropriate according to the reaction type. Specifically, the amount in terms of metal content is preferably 0.01 mass% or more, more preferably 0.05 mass% or more, and even more preferably 0.1 mass% or more, with respect to the amount of the compound (II) serving as a raw material, from the viewpoint of reactivity and economy and from the viewpoint of improving the yield, and is preferably 20 mass% or less, more preferably 10 mass% or less, and even more preferably 5 mass% or less, with respect to the amount of the compound (II), from the viewpoint of economy. The amount in terms of the metal content of the platinum group metal catalyst used is preferably 0.01 mass% or more and 20 mass% or less, more preferably 0.05 mass% or more and 10 mass% or less, and even more preferably 0.1 mass% or more and 5 mass% or less, with respect to the amount of the compound (II) serving as a raw material.

### <Simple Substance of Group 16 Element (Excluding Oxygen) or Compound Thereof>

It is considered that, in the present invention, a shorter reaction time and a higher reaction yield of the isomerization reaction are realized because a simple substance of a Group 16 element (excluding oxygen) or a compound thereof adsorbs on a platinum group metal catalyst and changes the electronic state of the metal surface, thereby stabilizing the adsorption state of α,β-unsaturated carbonyl on the metal surface, which reduces the activation energy of the isomerization reaction. That is to say, the simple substance of the Group 16 element (excluding oxygen) or the compound thereof is considered to work as a catalyst modifier.

The simple substance of the Group 16 element (excluding oxygen) and the compound thereof can be used together.

The Group 16 element includes one or more selected from sulfur, selenium, and tellurium. Among these, the simple substance of the Group 16 element (excluding oxygen) or the compound thereof preferably includes one or more selected from the group consisting of sulfur and a sulfur compound, selenium and a selenium compound, and tellurium and a tellurium compound, and more preferably includes sulfur.

The sulfur compound is preferably a compound having a sulfide group, a thiophene ring, or a thiol group. Examples thereof include: sulfide compounds having a hydrocarbon group having 1 to 18 carbon atoms, such as dodecyl sulfide (didodecyl sulfide) or methyl dodecyl sulfide; alkyl thiophene and poly alkyl thiophene; and thiol compounds having a hydrocarbon group having 1 to 18 carbon atoms, such as dodecylthiol (1-dodecanethiol).

Among these, the simple substance of the Group 16 element (excluding oxygen) or the compound thereof is preferably sulfur or a sulfur compound from the viewpoint of availability, ease of handling, reactivity, and the like, and more preferably sulfur from the viewpoint of recoverability and reusability. Sulfur is also referred to as sulfur as a simple substance. Furthermore, the use of sulfur as the simple substance of the Group 16 element (excluding oxygen) or the compound thereof is preferable because the reaction efficiency is almost maintained even if the platinum group metal catalyst is reused.

The simple substance of the Group 16 element (excluding oxygen) or the compound thereof is preferably a substance that can shift a spectral peak of a Pd 3d orbit toward the higher energy side by 0.1 to 1.0 eV, in the catalyst after use when compared with that before use, in X-ray electron spectroscopy (XPS or ESCA). For example, if sulfur as a simple substance is used in an amount of 13 mol% with respect to the amount of Pd, Pd 3d peaks of Pd/C shift from 335.5 eV and 340.8 eV to 335.9 eV and 341.1 eV

The measurement conditions for the X-ray electron spectroscopy are as follows.
Equipment: PHI Quantera SXM (ULVAC PHI Inc.)
Radiation source: Monochromatized Al Kα (1486.6 eV), output 25 W, 15 kV
Beam diameter: 100 pm Spot analysis
Pass energy: 280.0 eV (survey) 112.0 eV (narrow)
Step: 1.00 eV (survey) 0.20 eV (narrow)
Charge correction: Neutralizer and Ar
Photoelectron extraction angle: 45°

The amount of the simple substance of the Group 16 element (excluding oxygen) or the compound thereof used is preferably 100 mol% or less, more preferably 60 mol% or less, and even more preferably 40 mol% or less, with respect to the metal content of the platinum group metal catalyst used, from the viewpoint of isomerization yield, and is preferably 0.1 mol% or more, more preferably 0.5 mol% or more, and even more preferably 1 mol% or more, with respect to the metal content of the platinum group metal catalyst used, from the viewpoint of isomerization yield. The amount of the simple substance of the Group 16 element (excluding oxygen) or the compound thereof used is preferably 0.1 mol% or more and 100 mol% or less, more preferably 0.5 mol% or more and 60 mol% or less, and even more preferably 1 mol% or more and 40 mol% or less, with respect to the metal content of the platinum group metal catalyst used. The amount of the simple substance of the Group 16 element (excluding oxygen) or the compound thereof used is the total amount of the simple substance of the Group 16 element (excluding oxygen) and the compound thereof.

The isomerization reaction can be caused to occur, for example, at 80 to 250°C. The temperature of the isomerization reaction is preferably 100 to 200°C, and more preferably 120 to 160°C, from the viewpoint of reactivity and selectivity.

The isomerization reaction can proceed under atmospheric pressure, but the reaction can be caused to efficiently proceed under reduced pressure. The reaction pressure is preferably in the range of 20 to 200 kPa, and more preferably in the range of 50 to 150 kPa, according to the reaction temperature.

### <Solvent>

The present invention can be implemented in the presence or absence of a solvent. The absence of a solvent is advantageous from the viewpoint of productivity and economy. The solvent is not particularly limited, but can be an inert organic solvent, for example: alcohols such as methanol, ethanol, propanol, isopropanol, isobutanol, tert-butanol, n-butanol, 2-butanol, isopentanol, pentanol, hexanol, 2-ethylbutanol, heptanol, 2-heptanol, octanol, 2-octanol, cyclopentanol, cyclohexanol, ethylene glycol, propylene glycol, ethylene glycol monoethyl ether, ethylene glycol monopropyl ether, propylene glycol monoethyl ether, diethylene glycol, diethylene glycol monomethyl ether, benzyl alcohol, and phenylethanol; ketones such as methyl ethyl ketone, methyl isopropyl ketone, methyl propyl ketone, methyl isobutyl ketone, methyl n-butyl ketone, methyl n-amyl ketone, methyl isoamyl ketone, ethyl butyl ketone, methyl n-hexyl ketone, dipropyl ketone, diisobutyl ketone, cyclopentanone, and cyclohexanone; ethers such as isopropyl ether, n-butyl ether, 1,4-dioxane, isoamyl ether, n-hexyl ether, tetrahydropyran 2-methyl furan, diethylene glycol diethyl ether, methyl phenyl ether, and ethyl phenyl ether; esters such as n-methyl formate, n-propyl formate, n-butyl formate, methyl acetate, isopropyl acetate, n-butyl acetate, n-amyl acetate, n-hexyl acetate, cyclohexyl acetate, ethyl propionate, n-butyl propionate, methyl butyrate, n-butyl butyrate, methyl isovalerate, ethyl lactate, methyl benzoate, propyl benzoate, dimethyl phthalate, diethyl oxalate, dimethyl succinate, dimethyl glutarate, and dimethyl adipate; and hydrocarbons such as n-hexane, n-octane, n-decane, ligroin, cyclohexane, benzene, toluene, xylene, ethylbenzene, isopropyl benzene, amyl benzene, t-butylbenzene, p-cymene, tetralin, and decalin. These solvents can be used alone or in a combination of two or more.

The amount of solvent used is preferably 0.1 to 5 times, and more preferably 0.3 to 2 times the mass of the compound (II) serving as a raw material.

### [Step of Obtaining Compound of Formula (II)]

As described above, the present invention is directed to the method for producing a compound represented by General Formula (I), further including a step of dehydrating the compound represented by General Formula (III) above in the presence of an acid, thereby obtaining the compound represented by General Formula (II) above.

In the dehydrating step of the method, the presence of the molecular hydrogen and/or the hydrogen source, the acid, the platinum group metal catalyst, and the simple substance of the Group 16 element (excluding oxygen) or the compound thereof is preferable from the viewpoint of causing the dehydration reaction and the isomerization reaction in succession.

That is to say, the method for producing a compound represented by General Formula (I) preferably further includes a step of dehydrating the compound represented by General Formula (III) above in the presence of the molecular hydrogen and/or the hydrogen source, the acid, the platinum group metal catalyst, and the simple substance of the Group 16 element (excluding oxygen) or the compound thereof, thereby obtaining the compound represented by General Formula (II) above.

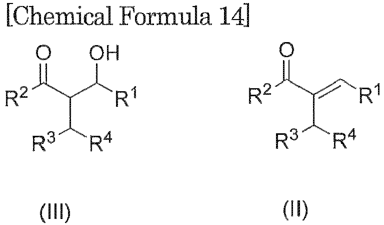

In the formulas, R¹, R², and R⁴ simultaneously or independently represent a hydrogen atom, a linear or branched alkyl group having 1 to 8 carbon atoms, or a linear or branched alkenyl group having 2 to 8 carbon atoms, and
R³ represents a linear or branched alkyl group having 1 to 8 carbon atoms or a linear or branched alkenyl group having 2 to 8 carbon atoms, or
R¹ and R⁴ simultaneously or independently represent a hydrogen atom, a linear or branched alkyl group having 1 to 8 carbon atoms, or a linear or branched alkenyl group having 2 to 8 carbon atoms, and
R² and R³, taken together, represent an alkanediyl group having 2 to 9 carbon atoms or represent an alkanediyl group having 2 to 9 carbon atoms substituted with one or two alkyl groups having 1 to 5 carbon atoms.

### <Molecular Hydrogen and/or Hydrogen Source>

The molecular hydrogen and/or the hydrogen source are as described above. If the molecular hydrogen and the inert gas are used in a mixed state in this step, the mixing ratio (volume ratio, molecular hydrogen / inert gas) is preferably 1/10 or more, more preferably 1/5 or more, and even more preferably 1/3 or more, from the viewpoint of reactivity, is preferably 10/1 or less, more preferably 5/1 or less, and even more preferably 3/1 or less, from the viewpoint of suppressing side reactions, and is preferably 10/1 to 1/10, more preferably 5/1 to 1/5, and even more preferably 3/1 to 1/3.

### <Platinum Group Metal Catalyst>

The platinum group metal catalyst is as described above. The amount of platinum metal catalyst used can be optimized as appropriate according to the reaction type. Specifically, the amount in terms of a metal content is preferably 0.01 mass% or more, more preferably 0.05 mass% or more, and even more preferably 0.1 mass% or more, with respect to the amount of the compound (III) serving as a raw material, from the viewpoint of reactivity and economy and from the viewpoint of improving the yield, and is preferably 20 mass% or less, more preferably 10 mass% or less, and even more preferably 5 mass% or less, with respect to the amount of the compound (III), from the viewpoint of economy. The amount in terms of a metal content of the platinum group metal catalyst used is preferably 0.01 mass% or more and 20 mass% or less, more preferably 0.05 mass% or more and 10 mass% or less, and even more preferably 0.1 mass% or more and 5 mass% or less, with respect to the amount of the compound (III) serving as a raw material.

### <Simple Substance of Group 16 Element (Excluding Oxygen) or Compound Thereof>

The simple substance of the Group 16 element (excluding oxygen) or the compound thereof is as described above. The amount of the simple substance of the Group 16 element (excluding oxygen) or the compound thereof used is preferably 100 mol% or less, more preferably 60 mol% or less, and even more preferably 40 mol% or less, with respect to the metal content of the platinum group metal catalyst used, from the viewpoint of isomerization yield, and is preferably 0.1 mol% or more, more preferably 0.5 mol% or more, and even more preferably 1 mol% or more, with respect to the metal content of the platinum group metal catalyst used, from the viewpoint of isomerization yield. The amount of the simple substance of the Group 16 element (excluding oxygen) or the compound thereof used is preferably 0.1 mol% or more and 100 mol% or less, more preferably 0.5 mol% or more and 60 mol% or less, and even more preferably 1 mol% or more and 40 mol% or less, with respect to the metal content of the platinum group metal catalyst used. The amount of the simple substance of the Group 16 element (excluding oxygen) or the compound thereof used is the total amount of the simple substance of the Group 16 element (excluding oxygen) and the compound thereof.

### <Acid>

The acid for use in the present invention can be one or more selected from inorganic acids, organic acids, and solid acids. Solid acids are preferred from the viewpoint of separation and removal from the reaction mixture.

The inorganic acids and the organic acids can be commonly used acids. Specific examples thereof include: inorganic acids such as hydrochloric acid, sulfuric acid, phosphoric acid, and condensed phosphoric acids such as orthophosphoric acid, metaphosphoric acid, pyrophosphoric acid, and tripolyphosphoric acid; and organic acids such as acetic acid, oxalic acid, citric acid, maleic acid, fumaric acid, and malic acid.

Among these acids, acid dissociation constant (pKa) in the first dissociation at 25°C of 0 or more and preferably 0.5 or more are preferred from the viewpoint of suppressing corrosion of the metal, and specific examples thereof include phosphoric acid (pKa in 1^{st} dissociation: 2.15), condensed phosphoric acids such as pyrophosphoric acid (pKa in 1^{st} dissociation: 0.8) and tripolyphosphoric acid, and organic acids such as acetic acid (pKa in 1^{st} dissociation: 4.56), oxalic acid (pKa in 1^{st} dissociation: 1.04), citric acid (pKa in 1^{st} dissociation: 2.87), maleic acid (pKa in 1^{st} dissociation: 1.75), fumaric acid (pKa in 1^{st} dissociation: 2.85), and malic acid (pKa in 1^{st} dissociation: 3.24).

Publicly known solid acids can be used as solid acid. Specific examples thereof include activated carbon, inorganic metal solids such as activated alumina, zirconia sulfate, metal phosphate, aluminumdihydrogen tripolyphosphate, titanium oxide, and the like, cation exchange resins, silica-titania composite oxides, silica-calcium oxide composite oxides, silica-magnesia composite oxides, and zeolite.

The solid acids are preferably those in which the amount (mmol/g) of acid sites that cause desorption of ammonia (NH₃) in the temperature range of 100 to 250°C is larger than the amount (mmol/g) of acid sites that cause desorption of NH₃ at a temperature higher than 250°C in the ammonia temperature programmed desorption (TPD). It is more preferable that the amount of acid sites that cause desorption of NH₃ in the range of 100 to 250°C is 0.01 mmol/g or more and that the amount of acid sites that cause desorption of NH₃ at a temperature higher than 250°C is less than 0.3 mmol/g. For example, phosphoric acid-supported activated carbon described in Examples below is a material in which the amount of acid sites that cause desorption of NH₃ in the range of 100 to 250°C in the TPD is 0.02 mmol/g.

The amount of acid sites described above is measured as follows: the high peak (the peak on the higher temperature side of the two observed peaks) of zeolite; JRC-Z5-25H, which is the Reference Catalyst of the Catalysis Society of Japan, is taken as 0.99 mmol/g, and the amount of acid sites is measured as the amount relative to the high peak. The peak is detected by quantifying ammonia with the m/e = 16 fragment of ammonia in the mass spectrum.

The TPD (ammonia temperature programmed desorption) measurement method can be commonly used measurement methods. For example, TPD measurement is performed after pretreatment, NH₃ adsorption treatment, and vacuum treatment are sequentially performed under the following conditions. Pretreatment: The temperature is increased to 200°C in helium in 20 minutes and held for 1 hour.
NH₃ adsorption treatment: NH₃ is adsorbed at 50°C and 2.7 kPa for 10 minutes. Vacuum treatment: The treatment is performed at 50°C for 4 hours.
TPD measurement: The temperature is increased to 600°C at a programming rate of 5°C/min while helium gas is circulated at 50 ml/min.

Solid acids having such a distribution of acid sites are, for example, preferably those having at least one of a structure (A), a structure (B), and metal atoms (C) below, and more preferably those having the structure (A) and the metal atoms (C), those having the structure (B) and the metal atoms (C), and those having the structure (A), the structure (B), and the metal atoms (C). The acid sites of solid acids are measured as a whole through the ammonia temperature programmed desorption (TPD). Specifically, the overall acid sites can be adjusted to a desired value by adjusting each of the structure (A), the structure (B), and the structure (C). Furthermore, it is also possible to support the structure (A) and the structure (B) on a carrier. In this case, the overall acid sites including those of the carrier can be adjusted to a desired value.
- Structure (A): Structure obtained by removing a hydrogen atom from at least one OH group of an inorganic phosphoric acid
- Structure (B): Structure obtained by removing a hydrogen atom from at least one OH group of an organic phosphoric acid
- Metal atoms (C): Atoms of one or more metals selected from aluminum, gallium, and iron

The structure (A) may refer to condensed phosphoric acids such as orthophosphoric acid, metaphosphoric acid, or pyrophosphoric acid, phosphoric acid, or the like. Among these, orthophosphoric acid and phosphoric acid are preferred from the viewpoint of performance.

The structure (B) may refer to phosphonic acid, phosphonic acid monoester, phosphinic acid, phosphoric acid monoester, phosphate diester, phosphite monoester, phosphite diester, or the like. Among these, phosphonic acid is preferred.

The metal atoms (C) are preferably aluminum from the viewpoint of performance and/or cost.

A small amount of metal atoms other than aluminum, gallium, and iron can be present to improve selectivity and other performance. Not all of the metal atoms (C) contained in the catalyst need to be bonded to the structure (A) or the structure (B), and some of the metal atoms (C) can be present in the form of metal oxides or metal hydroxides.

The solid acids can be prepared through precipitation, impregnation of metal oxides or hydroxides with inorganic or organic phosphoric acid, or replacement of inorganic phosphoric acid groups in inorganic aluminum phosphate gels with organic phosphoric acid groups, or the like. Among these, precipitation is preferred.

It is also possible to prepare a supported catalyst by coexisting a support with a large surface area in preparation of the solid acids. Silica, alumina, silica alumina, titania, zirconia, diatomaceous earth, activated carbon, or the like can be used as the support. The percentage of the support in the catalyst is preferably 90 wt% or less because the use of an excess amount of the support will reduce the content of active components and lower the activity.

The solid acids can be in powder or molded form. The solid acids can all have the same composition, or can be a combination of solid acids with different compositions.

The above-described inorganic acids, organic acids, and solid acids can be used alone or in a combination of two or more. When only solid acids are used, the neutralization step can be omitted.

The amount of solid acids used is preferably 0.0001 mass% or more with respect to the amount of the compound of Formula (III), from the viewpoint of reactivity. On the other hand, the amount of solid acids used is preferably 25 mass% or less, from the viewpoint of suppressing polymerization between compounds containing generated double bonds and improving the yield. From these points of view, the amount of solid acids used is more preferably 0.001 to 12 mass%, and even more preferably 0.01 to 6 mass%.

This step can be carried out, for example, at 80 to 250°C. The temperature of the isomerization reaction is preferably 120 to 200°C, and more preferably 120 to 160°C, from the viewpoint of reactivity and selectivity.

This step can proceed under atmospheric pressure, but the reaction can be caused to efficiently proceed under reduced pressure because water generated can be efficiently distilled out of the system and the raw materials and reaction products can be prevented from being distilled off. The reaction pressure is preferably in the range of 20 to 200 kPa, and more preferably in the range of 50 to 150 kPa, according to the reaction temperature. In the method of the present invention, it is preferable to cause the reaction to occur while distilling generated water out of the system.

### <Solvent>

The solvent is as described above. The amount of solvent used is preferably 0.1 to 5 times, and more preferably 0.3 to 2 times the mass of the compound (III) serving as a raw material.

### <Production of Compound of Formula (III)>

The compound of Formula (III) serving as a raw material can be produced using known methods. For example, it can be obtained by reacting a ketone represented by General Formula (X) below with an aldehyde represented by General Formula (XI). The ketone represented by General Formula (X) and the aldehyde represented by General Formula (XI) are commercially available or can be produced using known methods.

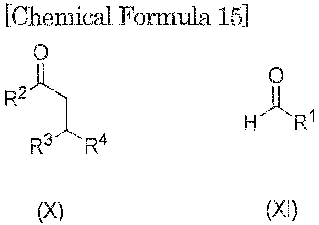

In the formulas, R¹, R², and R⁴ simultaneously or independently represent a hydrogen atom, a linear or branched alkyl group having 1 to 8 carbon atoms, or a linear or branched alkenyl group having 2 to 8 carbon atoms, and
R³ represents a linear or branched alkyl group having 1 to 8 carbon atoms or a linear or branched alkenyl group having 2 to 8 carbon atoms, or
R¹ and R⁴ simultaneously or independently represent a hydrogen atom, a linear or branched alkyl group having 1 to 8 carbon atoms, or a linear or branched alkenyl group having 2 to 8 carbon atoms, and
R² and R³, taken together, represent an alkanediyl group having 2 to 9 carbon atoms or represent an alkanediyl group having 2 to 9 carbon atoms substituted with one or two alkyl groups having 1 to 5 carbon atoms.

In the present invention, the compound (III) obtained using such a method can be used without purification, but can be produced through distillation or the like before use in the case in which the activity of the catalyst decreases, for example.

### [Method for Producing Compound of Formula (VII)]

The present invention is directed to a method for producing a compound of Formula (VII), including Steps 1 and 2 below.

Step 1: Step of dehydrating and isomerizing a compound represented by General Formula (IV) below in the presence of molecular hydrogen and/or a hydrogen source, an acid, a platinum group metal catalyst, and a simple substance of a Group 16 element (excluding oxygen) or a compound thereof, thereby obtaining a compound represented by General Formula (V) below

Step 2: Step of reacting the compound represented by General Formula (V) obtained in Step 1 with a malonic acid diester represented by General Formula (VI) below and then with water, thereby obtaining a compound represented by General Formula (VII) below

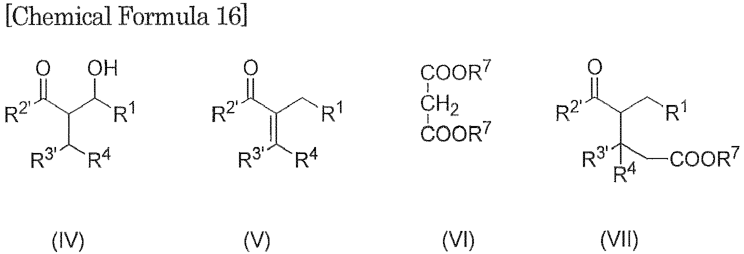

In the formulas, R¹ and R⁴ simultaneously or independently represent a hydrogen atom, a linear or branched alkyl group having 1 to 8 carbon atoms, or a linear or branched alkenyl group having 2 to 8 carbon atoms,

R²' and R³', taken together, represent an alkanediyl group having 2 to 9 carbon atoms or represent an alkanediyl group having 2 to 9 carbon atoms substituted with one or two alkyl groups having 1 to 5 carbon atoms, and

R⁷ represents an alkyl group having 1 to 3 carbon atoms, and two R⁷s are optionally the same or different.

The compound of General Formula (IV) (also referred to as a "compound of Formula (IV)" or a "compound (IV)" hereinafter) corresponds to the compound of Formula (III) in which the definitions of R² and R³ are limited. Furthermore, the compound of General Formula (V) (also referred to as a "compound of Formula (V)" or a "compound (I)" hereinafter) corresponds to the compound of Formula (V) in which the definitions of R² and R³ are limited.

### <Step 1>

In this producing method, Step 1, namely "a step of dehydrating and isomerizing a compound represented by General Formula (IV) below in the presence of molecular hydrogen and/or a hydrogen source, an acid, a platinum group metal catalyst, and a simple substance of a Group 16 element (excluding oxygen) or a compound thereof, thereby obtaining a compound represented by General Formula (V) below" is carried out according to the method for producing a compound represented by General Formula (I) above, that is, the method for producing a compound represented by General Formula (I), further including a step of dehydrating the compound represented by General Formula (III) above in the presence of the molecular hydrogen and/or the hydrogen source, the acid, the platinum group metal catalyst, and the simple substance of the Group 16 element (excluding oxygen) or the compound thereof, thereby obtaining the compound represented by General Formula (II) above. That is to say, the method is carried out according to the method for producing the compound of Formula (1), except that the compound of Formula (III) is replaced with the compound of Formula (IV) and the compound of Formula (I) is replaced with the compound of Formula (V).

### <Step 2>

Step 2 is a step of reacting the compound of Formula (V) obtained in Step 1 with a malonic acid diester represented by General Formula (VI) and then with water, thereby obtaining the compound represented by General Formula (VII) (also referred to as a "compound of Formula (VII)" or a "compound (VII)" hereinafter).

Specifically, first, a compound represented by General Formula (VIII) (also referred to as a "compound of Formula (VIII)" or a "compound (VIII)" hereinafter) is obtained by reacting the compound of Formula (V) with a malonic acid diester of Formula (VI) in the presence of a base.

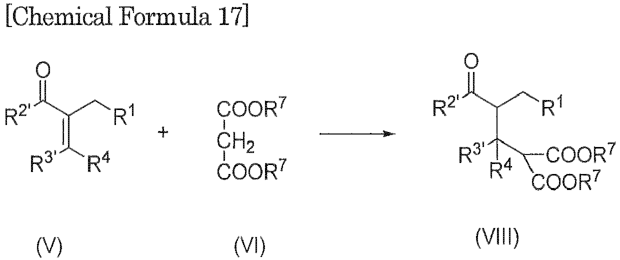

In the formulas, R¹, R²', R³', R⁴, and R⁷ are as defined above, and two R⁷s are optionally the same or different.

The compound (VII) is reacted in an amount of preferably 1 to 5 times, more preferably 1.2 to 2 times the amount in moles of the compound (V) serving as a raw material.

Examples of the base that can be used include alkali metals such as sodium and potassium, and alkali metal alkoxides such as sodium alkoxide and potassium alkoxide.

The amount of base used is preferably 0.005 to 0.2 times, and more preferably 0.01 to 0.1 times the amount in moles of the compound (V). Polar solvents such as alcohols are preferable as solvent.

The reaction temperature is preferably -10 to 30°C, and more preferably -2 to 20°C.

Next, the compound (VII) can be produced by reacting obtained compound (VIII) with water. The water is preferably added in an amount of 1 to 3 times the amount in moles of the compound (VIII), and the reaction is preferably caused to occur by adding the water dropwise to the reaction system. The reaction temperature at this time is preferably 150 to 230°C, and more preferably 180 to 220°C.

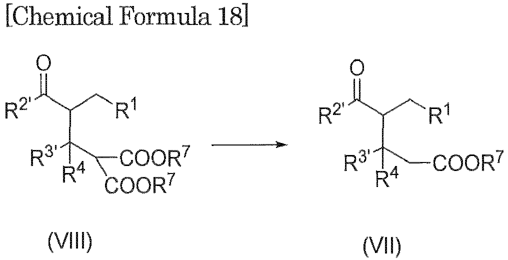

In the formulas, R¹, R²', R³', R⁴, and R⁷ are as defined above, and two R⁷s are optionally the same or different.

The thus obtained compound (VII) can be obtained in a higher yield and has fewer impurities compared with conventional production methods, and thus reducing the purification load to obtain the compound (VII) in high purity and making it an excellent fragrance material.

With respect to the foregoing embodiment, the present invention further discloses the following methods.
[1] A method for producing a compound represented by General Formula (I) below, including a step of isomerizing a compound represented by General Formula (II) below in the presence of molecular hydrogen and/or a hydrogen source, a platinum group metal catalyst, and a simple substance of a Group 16 element (excluding oxygen) or a compound thereof. 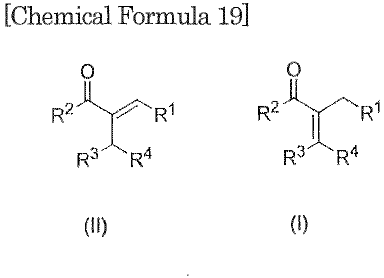 In the formulas, R¹, R², and R⁴ simultaneously or independently represent a hydrogen atom, a linear or branched alkyl group having 1 to 8 carbon atoms, or a linear or branched alkenyl group having 2 to 8 carbon atoms, and
   R³ represents a linear or branched alkyl group having 1 to 8 carbon atoms or a linear or branched alkenyl group having 2 to 8 carbon atoms, or
   R¹ and R⁴ simultaneously or independently represent a hydrogen atom, a linear or branched alkyl group having 1 to 8 carbon atoms, or a linear or branched alkenyl group having 2 to 8 carbon atoms, and
   R² and R³, taken together, represent an alkanediyl group having 2 to 9 carbon atoms or represent an alkanediyl group having 2 to 9 carbon atoms substituted with one or two alkyl groups having 1 to 5 carbon atoms.
[2] The producing method according to [1], wherein, in the formulas above, R² and R³, taken together, represent an alkanediyl group having 2 to 9 carbon atoms.
[3] The producing method according to [1] or [2], wherein a metal of the platinum group metal catalyst is one or more selected from the group consisting of osmium (Os), ruthenium (Ru), iridium (Ir), rhodium (Rh), platinum (Pt), and palladium (Pd).
[4] The producing method according to any one of [1] to [3], wherein a metal of the platinum group metal catalyst is palladium or platinum.
[5] The producing method according to any one of [1] to [4], wherein a metal of the platinum group metal catalyst is supported on carbon.
[6] The producing method according to any one of [1] to [5], wherein the amount of the platinum group metal catalyst used in terms of a metal content is 0.01 mass% or more and 20 mass% or less with respect to the amount of the compound (II) serving as a raw material.
[7] The producing method according to any one of [1] to [6], wherein the amount of the platinum group metal catalyst used in terms of a metal content is 0.05 mass% or more and 10 mass% or less with respect to the amount of the compound (II) serving as a raw material.
[8] The producing method according to any one of [1] to [7], wherein the amount of the platinum group metal catalyst used in terns of a metal content is 0.1 mass% or more and 5 mass% or less with respect to the amount of the compound (II) serving as a raw material.
[9] The producing method according to any one of [1] to [8], wherein the simple substance of the Group 16 element (excluding oxygen) or the compound thereof includes one or more selected from the group consisting of sulfur and a sulfur compound, selenium and a selenium compound, and tellurium and a tellurium compound.
[10] The producing method according to any one of [1] to [9], wherein the sulfur compound is a compound having a sulfide group, a thiophene ring, or a thiol group.
[11] The producing method according to any one of [1] to [10], wherein the simple substance of the Group 16 element (excluding oxygen) or the compound thereof includes sulfur.
[12] The producing method according to any one of [1] to [11], wherein the molecular hydrogen is mixed with inert gas, and the inert gas is one or more selected from the group consisting of nitrogen, argon, and helium.
[13] The producing method according to any one of [1] to [12], wherein a volume ratio of the molecular hydrogen to the inert gas (molecular hydrogen / inert gas) is 10/1 to 1/30.
[14] The producing method according to any one of [1] to [13], wherein a volume ratio of the molecular hydrogen to the inert gas (molecular hydrogen / inert gas) is 5/1 to 1/20.
[15] The producing method according to any one of [1] to [14], wherein a volume ratio of the molecular hydrogen to the inert gas (molecular hydrogen / inert gas) is 3/1 to 1/10.
[16] The producing method according to any one of [1] to [15], wherein the amount of the simple substance of the Group 16 element (excluding oxygen) or the compound thereof used is 0.1 mol% or more and 100 mol% or less with respect to a metal content of the platinum group metal catalyst.
[17] The producing method according to any one of [1] to [16], wherein the amount of the simple substance of the Group 16 element (excluding oxygen) or the compound thereof used is 0.5 mol% or more and 60 mol% or less with respect to a metal content of the platinum group metal catalyst.
[18] The producing method according to any one of [1] to [17], wherein the amount of the simple substance of the Group 16 element (excluding oxygen) or the compound thereof used is 1 mol% or more and 40 mol% or less with respect to a metal content of the platinum group metal catalyst.
[19] The producing method according to any one of [1] to [18], wherein the isomerization reaction is performed at 80 to 250°C.
[20] The producing method according to any one of [1] to [19], wherein the isomerization reaction is performed at 100 to 200°C.
[21] The producing method according to any one of [1] to [20], wherein the isomerization reaction is performed at 120 to 160°C.
[22] The producing method according to any one of [1] to [21], further including a step of dehydrating the compound represented by General Formula (III) above in the presence of the molecular hydrogen and/or the hydrogen source, an acid, the platinum group metal catalyst, and the simple substance of the Group 16 element (excluding oxygen) or the compound thereof, thereby obtaining the compound represented by General Formula (II) above. 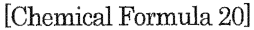 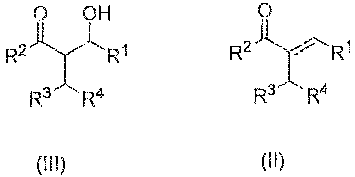 In the formulas, R¹, R², and R⁴ simultaneously or independently represent a hydrogen atom, a linear or branched alkyl group having 1 to 8 carbon atoms, or a linear or branched alkenyl group having 2 to 8 carbon atoms, and
   R³ represents a linear or branched alkyl group having 1 to 8 carbon atoms or a linear or branched alkenyl group having 2 to 8 carbon atoms, or
   R¹ and R⁴ simultaneously or independently represent a hydrogen atom, a linear or branched alkyl group having 1 to 8 carbon atoms, or a linear or branched alkenyl group having 2 to 8 carbon atoms, and
   R² and R³, taken together, represent an alkanediyl group having 2 to 9 carbon atoms or represent an alkanediyl group having 2 to 9 carbon atoms substituted with one or two alkyl groups having 1 to 5 carbon atoms.
[23] The producing method according to [22], wherein the acid includes a solid acid.
[24] The producing method according to [22] or [23], wherein the amount of solid acid used is 0.001 to 12 mass% with respect to the amount of the compound of Formula (III).
[25] The producing method according to any one of [22] to [24], wherein the amount of solid acid used is 0.01 to 6 mass% with respect to the amount of the compound of Formula (III).
[26] The producing method according to any one of [1] to [25], wherein, in the formulas above, R² and R³, taken together, are an alkanediyl group having 2 to 9 carbon atoms.
[27] The producing method according to any one of [1] to [26], wherein, in the formulas above, R² and R³, taken together, are an alkanediyl group having 2 to 4 carbon atoms.
[28] The producing method according to any one of [1] to [27], wherein, in the formulas above, R² and R³, taken together, are an alkanediyl group having 2 carbon atoms.
[29] The producing method according to any one of [1] to [28], wherein, in the formulas above, R¹ is an alkyl group having 1 to 4 carbon atoms.
[30] The producing method according to any one of [1] to [29], wherein, in the formulas above, R¹ is an n-butyl group.
[31] The producing method according to any one of [1] to [30], wherein, in the formulas above, R⁴ is a hydrogen atom.
[32] The producing method according to any one of [1] to [31], wherein, in the formulas above, R¹ is an n-butyl group, R² and R³, taken together, are an alkanediyl group having 2 carbon atoms, and R⁴ is a hydrogen atom.
[33] The producing method according to any one of [1] to [32], wherein the compound represented by General Formula (III) above is 2-(1-hydroxypentyl)-cyclopentan- 1-one.
[34] A method for producing a compound of Formula (VII), including Steps 1 and 2 below.

Step 1: Step of dehydrating and isomerizing a compound represented by General Formula (IV) below in the presence of molecular hydrogen and/or a hydrogen source, an acid, a platinum group metal catalyst, and a simple substance of a Group 16 element (excluding oxygen) or a compound thereof, thereby obtaining a compound represented by General Formula (V) below

Step 2: Step of reacting the compound represented by General Formula (V) obtained in Step 1 with a malonic acid diester represented by General Formula (VI) below and then with water, thereby obtaining a compound represented by General Formula (VII) below

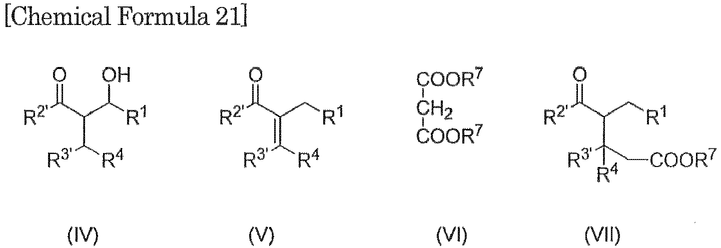

In the formulas, R¹ and R⁴ simultaneously or independently represent a hydrogen atom, a linear or branched alkyl group having 1 to 8 carbon atoms, or a linear or branched alkenyl group having 2 to 8 carbon atoms,
R²' and R³', taken together, represent an alkanediyl group having 2 to 9 carbon atoms or represent an alkanediyl group having 2 to 9 carbon atoms substituted with one or two alkyl groups having 1 to 5 carbon atoms, and
R⁷ represents an alkyl group having 1 to 3 carbon atoms, and two R⁷s are optionally the same or different.

[35] The producing method according to [34], wherein R²' and R³', taken together, are an alkanediyl group having 2 to 4 carbon atoms. The producing method according to claim 1.

[36] The producing method according to [34] or [35], wherein R²' and R³', taken together, are an alkanediyl group having 2 carbon atoms. The producing method according to claim 1.

[37] The producing method according to any one of [34] to [36], wherein R¹ is an alkyl group having 1 to 4 carbon atoms.

[38] The producing method according to any one of [34] to [37], wherein R¹ is an n-butyl group.

[39] The producing method according to any one of [34] to [38], wherein R⁴ is a hydrogen atom.

[40] The producing method according to any one of [34] to [39], wherein R⁷ is a methyl group.

[41] The producing method according to any one of [34] to [40], wherein R¹ is an n-butyl group, R²' and R³', taken together, are an alkanediyl group having 2 carbon atoms, R⁴ is a hydrogen atom, and R⁷ represents a methyl group.

In the following reference examples, examples, and comparative examples, "%" is "mass%" unless otherwise noted. Furthermore, the operating pressure is 101 lPa (atmospheric pressure) unless otherwise noted. The mass of the catalyst is the mass in a dry state.

### <Gas Chromatography Equipment and Analytical Conditions>

GC Equipment: Hydrogen flame ionization detector 7890 manufactured by Agilent Technologies, Inc.
Column: For yield analysis, DB-1 (capillary column, 100% dimethylpolysiloxane, inner diameter 0.25 mm, length 30 m, film thickness 0.25 µm, manufactured by Agilent Technologies, Inc.) was used.
Carrier gas: He, 1.6 mL/min
Injection conditions: 200°C, split ratio 100/1
Dosing volume: 1 pL
Detection conditions: FID method, 280°C

The analysis was performed by the internal standard method (internal standard: undecane (manufactured by Tokyo Chemical Industry Co., Ltd., purity 99%).

Column temperature conditions: Starting from 100°C, the temperature was increased to 210°C at a rate of 5°C/min. The temperature was then increased to 280°C at a rate of 20°C/min and held at 280°C for 4.5 minutes.

### Reference Example 1

### Production of 2-(1-hydroxypentyl)-cyclopentan-1-one (4)

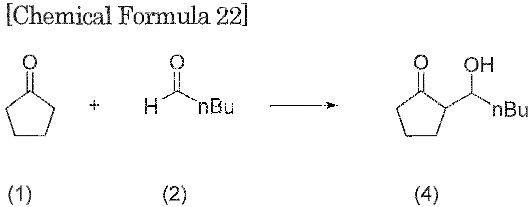

Cyclopentanone (1) (2241 Kg), water (1007 kg), and 48%NaOH (11 kg) were added to a 6-m³ reaction vessel having a dropping layer. After the mixture in the reaction vessel was cooled to 15°C with stirring, valeraldehyde (2) (985 kg) was added dropwise at the same temperature over 5 hours. After the dropwise addition was completed, the reaction mixture was stirred for 1 hour. The reaction mixture was neutralized and excess cyclopentanone was recovered from the mixture by distillation. The reaction end product (1868 kg) in the residual organic layer contained 1706 kg of 2-(1-hydroxypentyl)-cyclopentan-1-one (4). Note that 2-(1-hydroxypentyl)-cyclopentan-1-one (4) is a known compound, and its structure was confirmed in a publicly known literature such as JP 2009-269910A.

### Reference Example 2

### Production of 2-pentylcyclopentan-1-one (7)

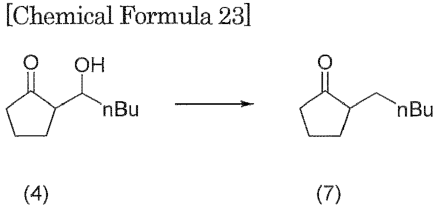

2-(1-Hydroxypentyl)-cyclopentan-1-one (4) (100 g, purity 88%) obtained in Reference Example 1, phosphoric acid-supported activated carbon (powder, 1.1 g), and 5% Pd/C (powder, water content 60.4%, 7.6 g) were added to a 200-mL four-necked flask (made of glass) equipped with a connecting tube, and the mixture was stirred at 400 rpm using a crescent blade (blade diameter 4 cm) and heated to 140°C and 101 kPa in an atmosphere of nitrogen : hydrogen (volume ratio) = 1: 0.33. During the reaction, the distillate was continuously distilled into a distillate receiver connected to the above-mentioned flask.

After 11 hours from the start of the reaction, 15.4 g of distillate was obtained and 93.3 g of reaction end product was obtained. Quantification of the reaction end product using GC showed that 2-pentyl-2-cyclopenten-1-one (3) (71.2 g) was produced and 2-pentylcyclopentan-1-one (7) (2.7 g) was formed as a byproduct. The yield of 2-pentyl-2-cyclopenten-1-one (3) was 89 mol%, and the formation rate of 2-pentylcyclopentan-1-one (7) was 8 mol%. Note that 2-pentylcyclopentan-1-one (7) is a known compound, and its structure was confirmed in a publicly known literature such as JP 2009-269910A.

### <Effect of Adding Sulfur>

### Example 1

2-(1-Hydroxypentyl)-cyclopentan-1-one (4) (100 g, purity 80%) obtained in Reference Example 1, phosphoric acid-supported activated carbon (powder, 1.1 g, 1.3 mass% with respect to the amount of the compound (4)), 5% Pd/C (powder, water content 60.4%, 7.6 g, 3.7 mass% with respect to the amount of the compound (4)), and a sulfur powder (manufactured by Fujifilm Wako Pure Chemical Corporation, powder, 2.2 mg, 4 mol% with respect to the metal content of the platinum group metal catalyst) were added to a 200-mL four-necked flask (made of glass) equipped with a connecting tube, and the mixture was stirred at 400 rpm using a crescent blade (blade diameter 4 cm) and heated to 140°C and 101 kPa in an atmosphere of nitrogen : hydrogen (volume ratio) = 1 : 0.33. During the reaction, the distillate was continuously distilled into a distillate receiver connected to the above-mentioned flask.

After 8 hours from the start of the reaction, 15.2 g of distillate was obtained and 93.5 g of reaction end product was obtained. Quantification of the reaction end product using GC showed that 2-pentyl-2-cyclopenten-1-one (3) (56.4 g) was produced and 2-pentylcyclopentan-1-one (7) (1.4 g) was formed as a byproduct. The yield of 2-pentyl-2-cyclopenten-1-one (3) was 76%, and the formation rate of 2-pentylcyclopentan-1-one (7) was 2%.

### Examples 2 to 4

The procedure was the same as that for Example 1, except that the amount of sulfur powder was changed to those shown in Table 1. Table 1 shows the yields of 2-pentyl-2-cyclopenten-1-one (3) obtained and the formation rates of 2-pentylcyclopentan-1-one (7).

### Comparative Example 1

The procedure was the same as that for Example 1, except that the sulfur powder was not used. Table 1 shows the yield of 2-pentyl-2-cyclopenten-1-one (3) obtained and the formation rate of 2-pentylcyclopentan-1-one (7).

**Table 1**

| 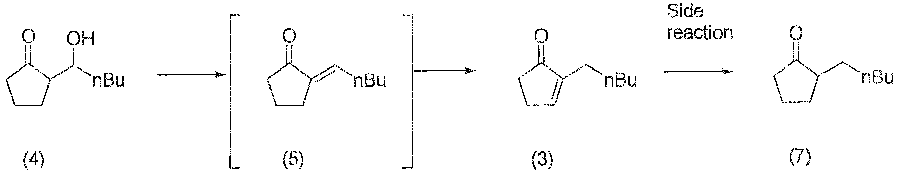 | | | | | |
|---|---|---|---|---|---|
| | Ex. 1 | Ex. 2 | Ex. 3 | Ex. 4 | Com.Ex. 1 |
| Solid acid (mass% with respect to Compound (4)) | Phosphoric acid-supported activated carbon (1.3 mass%) | | | | |
| Platinum group metal catalyst (mass% with respect to Compound (4)) | 5% Pd/C (3.7 mass%) | | | | |
| Nitrogen : Hydrogen | 1.0 : 0.33 | | | | |
| Simple substance of Group 16 element (mol% with respect to metal content of platinum group metal catalyst) | Sulfur powder (4 mol%) | Sulfur powder (13 mol%) | Sulfur powder (22 mol%) | Sulfur powder (33 mol%) | - |
| Reaction temperature | 140°C | | | | |
| Reaction time (hours) | 8 | | | | |
| Yield (%) of Compound (3) | 76% | 95% | 94% | 68% | 53% |
| Formation rate (%) of Compound (7) | 2% | 3% | 3% | 2% | 1% |

It is seen from Table 1 that the yield of isomerization from the compound (4) to the compound (3) is improved by using a sulfur powder as a simple substance of a Group 16 element.

### Example 5

2-(1-Hydroxypentyl)-cyclopentan-1-one (4) (306 g, purity 80%) obtained in Reference Example 1, phosphoric acid-supported activated carbon (powder, 3.3 g, 1.3 mass% with respect to the amount of the compound (4)), 5% Pd/C (powder, water content 61.7%, 7.83 g, 1.2 mass% with respect to the amount of the compound (4)), and a sulfur powder (manufactured by Fujifilm Wako Pure Chemical Corporation, powder, 6.3 mg, 13 mol% with respect to the metal content of the platinum group metal catalyst) were added to a 500-mL four-necked separable flask (made of glass) equipped with a connecting tube, and the mixture was stirred at 400 rpm using a 6-bladed disk turbine blade (blade diameter 3 cm) and heated and mixed to 101 kPa, 140°C in an atmosphere of nitrogen : hydrogen (volume ratio) = 1 : 1.5. During the reaction, the distillate was continuously distilled into a distillate receiver connected to the above-mentioned flask. After the reaction for 8 hours, 36.7 g of distillate was obtained and 268.6 g of reaction end product was obtained. The obtained reaction end product was quantified using GC. Table 2 shows the yield of 2-pentyl-2-cyclopenten-1-one (3) obtained and the formation rate of 2-pentylcyclopentan-1-one (7).

### Example 6

The procedure was the same as that for Example 5, except that the amount of 5% Pd/C was changed to 3.91 g (powder, water content 61.7%, 0.6 mass% with respect to the amount of the compound (4)). Table 2 shows the yields of 2-pentyl-2-cyclopenten-1-one (3) and the formation rates of 2-pentylcyclopentan-1-one (7), which were obtained after the reaction for 8 hours and after the reaction for 12 hours.

### Comparative Example 2

In this example, 2-(1-hydroxypentyl)-cyclopentan-1-one (4) (299 g, purity 89%) obtained in Reference Example 1, phosphoric acid-supported activated carbon (powder, 3.3 g, 1.2 mass% with respect to the amount of the compound (4)), 5% Pd/C (powder, water content 61.7%, 7.86 g, 1.1 mass% with respect to the amount of the compound (4)) were added to a 500-mL four-necked separable flask (made of glass) equipped with a connecting tube, and the mixture was stirred at 400 rpm using a 6-bladed disk turbine blade (blade diameter 3 cm) and heated and mixed to 140°C and 101 kPa in an atmosphere of nitrogen : hydrogen (volume ratio) = 1 : 1.5. During the reaction, the distillate was continuously distilled into a distillate receiver connected to the above-mentioned flask.

The reaction end products excluding the distillate obtained after the reaction for 8 hours and after the reaction for 18 hours were quantified using GC. Table 2 shows the yields of 2-pentyl-2-cyclopenten-1-one (3) obtained and the formation rates of 2-pentylcyclopentan-1-one (7).

**Table 2**

| 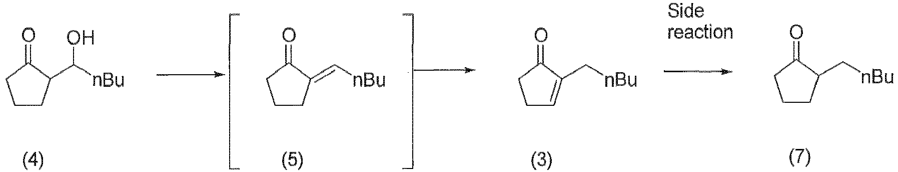 | | | | | |
|---|---|---|---|---|---|
| | Ex. 5 | Ex. 6 | | Com.Ex. 2 | |
| Solid acid (mass% with respect to Compound (4)) | Phosphoric acid-supported. activated carbon (1.3 mass%) | | | Phosphoric acid-supported activated carbon (1.2 mass%) | |
| Platinum group metal catalyst (mass% with respect to Compound (4)) | 5% Pd/C (1.2 mass%) | 5% Pd/C (0.6 mass%) | 5% Pd/C (0.6 mass%) | 5% Pd/C (1.1 mass%) | 5% Pd/C (1.1 mass%) |
| Nitrogen: Hydrogen | 1.0 : 1.5 | | | | |
| Simple substance of Group 16 element (mol% with respect to metal content of platinum group metal catalyst) | Sulfur powder (13 mol%) | | | - | |
| Reaction temperature | 140°C | | | | |
| Reaction time (hours) | 8 | 8 | 12 | 8 | 18 |
| Yield (%) of Compound (3) | 88 | 51 | 86 | 19 | 86 |
| Formation rate (%) of Compound (7) | 8 | 5 | 10 | 1 | 11 |

It is seen from comparison between Example 5 and Comparative Example 2 having substantially the same amount of metal catalyst that the reaction time can be shortened. Also, it is seen from comparison between Example 6 and Comparative Example 2 that the amount of metal catalyst can be reduced and the reaction time can be shortened.

### <Effect of Amount of Molecular Hydrogen>

### Example 7

2-(1-Hydroxypentyl)-cyclopentan-1-one (4) (301 g, purity 88%) obtained in Reference Example 1, phosphoric acid-supported activated carbon (powder, 3.3 g, 1.2 mass% with respect to the amount of the compound (4)), 5% Pd/C (powder, water content 60.9%, 7.67 g, 1.1 mass% with respect to the amount of the compound (4)), and a sulfur powder (manufactured by Fujifilm Wako Pure Chemical Corporation, powder, 7.0 mg, 13 mol% with respect to the metal content of the platinum group metal catalyst) were added to a 500-mL four-necked separable flask (made of glass) equipped with a connecting tube, and the mixture was stirred at 400 rpm using a 6-bladed disk turbine blade (blade diameter 3 cm) and heated and mixed to 140°C and 101 kPa in an atmosphere of nitrogen : hydrogen (volume ratio) = 1:3. During the reaction, the distillate was continuously distilled into a distillate receiver connected to the above-mentioned flask.

After 8 hours from the start of the reaction, 38.2 g of distillate was obtained and 273.8 g of reaction end product was obtained. Table 3 shows the quantification results of the reaction end product performed using GC.

**Table 3**

| 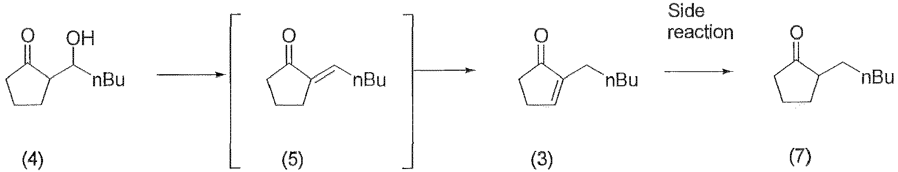 | | |
|---|---|---|
| | Ex. 5 | Ex. 7 |
| Solid acid (mass% with respect to Compound (4)) | Phosphoric acid-supported activated carbon (1.2 mass%) | |
| Platinum group metal catalyst (mass% with respect to Compound (4)) | 5% Pd/C (1.2 mass%) | 5% Pd/C (1.1 mass%) |
| Nitrogen : Hydrogen | 1 : 1.5 | 1:3 |
| Simple substance of Group 16 element (mol% with respect to metal content of platinum group metal catalyst) | Sulfur powder (13 mol%) | Sulfur powder (13 mol%) |
| Reaction temperature | 140°C | |
| Reaction time (hours) | 8 | |
| Yield (%) of Compound (3) | 88 | 78 |
| Formation rate (%) of Compound (7) | 8 | 17 |

It is seen from comparison between Examples 5 and 7 that the yield of the compound (3) was higher in Example 5 in which nitrogen : hydrogen (volume ratio) = 1:1.5.

### <Effects of Type of Compound of Group 16 Element>

### Examples 8 and 9

The procedure was the same as that for Example 6, except that the type of compound of a Group 16 element was changed to those shown in Table 4. Table 4 shows the yields of 2-pentyl-2-cyclopenten-1-one (3) and the formation rates of 2-pentylcyclopentan-1-one (7), which were obtained after the reaction for 8 hours and after the reaction for 16 hours.

**Table 4**

| 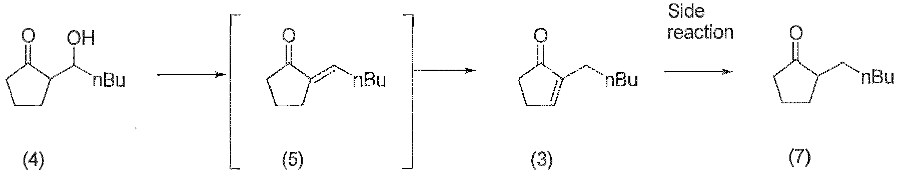 | | | | | | |
|---|---|---|---|---|---|---|
| | Ex. 6 | | Ex. 8 | | Ex. 9 | |
| Solid acid (mass% with respect to Compound (4)) | Phosphoric acid-supported activated carbon (1.3 mass%) | | | | | |
| Platinum group metal catalyst (mass% with respect to Compound (4)) | 5% Pd/C (0.6 mass%) | | | | | |
| Nitrogen : Hydrogen | 1:1.5 | | | | | |
| Simple substance of Group 16 element or compound thereof (mol% with respect to metal content of platinum group metal catalyst) | Sulfur powder (1.3 mol%) | | Dodecyl sulfide (13 mol%) | | Dodecanethiol (13 mol%) | |
| Reaction temperature | 140°C | | | | | |
| Reaction time (hours) | 8 | 12 | 8 | 16 | 8 | 16 |
| Yield (%) of Compound (3) | 51 | 86 | 53 | 89 | 49 | 89 |
| Formation rate (%) of Compound (7) | 5 | 10 | 2 | 4 | 2 | 5 |

It is seen from the results of Examples 6, 8, and 9 that a sulfur powder, dodecyl sulfide, and dodecanethiol are similarly effective as a simple substance of a Group 16 element or a compound thereof.

### <Isomerization from Compound (5) to Compound (3)>

### Reference Example 3

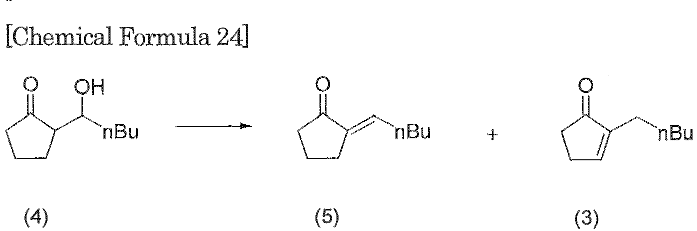

2-(1-Hydroxypentyl)-cyclopentan-1-one (4) (703 g, purity 86%) obtained in Reference Example 1 and phosphoric acid-supported activated carbon (powder, 21.0 g) were added to a 1000-mL autoclave (made of SUS) equipped with a connecting tube, and the mixture was heated and mixed to 140°C and 101 kPa in a nitrogen atmosphere. During the reaction, the distillate was continuously distilled into a distillate receiver connected to the above-mentioned autoclave.

After 2 hours from the start of the reaction, the reaction mixture excluding the distillate was cooled, and phosphoric acid-activated activated carbon was removed by filtration. The obtained filtrate was purified by distillation. The obtained 459.5 g of distilled distillate contained 2-pentyl-2-cyclopenten-1-one (3) (24.3 g) and 2-pentylidenecyclopentan-1-one (5) (407.6 g). Note that 2-pentylidenecyclopentan-1-one (5) is a known compound, and its structure was confirmed in publicly known literature such as Synthesis 1981; 1981(12): 1003-1004.

### Example 10

100 g of Distilled distillate (2-pentylidenecyclopentan-1-one (5) (purity 90%) and 2-pentyl-2-cyclopenten-1-one (3) (purity 5%)) obtained in Reference Example 3, 5% Pd/C (powder, water content 59.9%, 2.5 g, 1.1 mass% with respect to the amount of the compound (5)), and a sulfur powder (manufactured by Fujifilm Wako Pure Chemical Corporation, powder, 2.6 mg, 13 mol% with respect to the metal content of the platinum group metal catalyst) were added to a 200-mL four-necked flask (made of glass) equipped with a connecting tube, and the mixture was stirred at 400 rpm using a crescent blade (blade diameter 4 cm) and heated to 140°C and 101 kPa in an atmosphere of nitrogen : hydrogen (volume ratio) = 1 : 1.5. During the reaction, the distillate was continuously distilled into a distillate receiver connected to the above-mentioned flask.

After the reaction for 2 hours, 0.3 g of distillate was obtained and 102.2 g of reaction end product was obtained. Quantification of the reaction end product using GC showed that 2-pentyl-2-cyclopenten-1-one (3) (93.5 g) was produced and 2-pentylcyclopentan-1-one (7) (2.0 g) was formed as a byproduct. The yield of 2-pentyl-2-cyclopenten-1-one (3) was 98 mol%, and the formation rate of 2-pentylcyclopentan-1-one (7) was 2 mol%.

### Comparative Example 3

100 g of Distilled distillate (2-pentylidenecyclopentan-1-one (5) (purity 90%) and 2-pentyl-2-cyclopenten-1-one (3) (purity 5%)) obtained in Reference Example 3 and 5% Pd/C (powder, water content 59.9%, 7.5 g, 3.3 mass% with respect to the amount of the compound (5)) were added to a 200-mL four-necked flask (made of glass) equipped with a connecting tube, and the mixture was stirred at 400 rpm using a crescent blade (blade diameter 4 cm) and heated to 140°C and 101 kPa in an atmosphere of nitrogen : hydrogen (volume ratio) = 1 : 1.5. During the reaction, the distillate was continuously distilled into a distillate receiver connected to the above-mentioned flask. After the reaction for 4 hours, 4.0 g of distillate was obtained and 103.5 g of reaction end product was obtained. The obtained reaction end product was quantified using GC. Table 5 shows the yield of 2-pentyl-2-cyclopenten-1-one (3) obtained and the formation rate of 2-pentylcyclopentan-1-one (7).

### Comparative Example 4

The procedure was the same as that for Comparative Example 4, except that the amount of 5% Pd/C was changed to 2.5 g (1.1 mass% with respect to the amount of the compound (5)). Table 5 shows the yield of 2-pentyl-2-cyclopenten-1-one (3) and the formation rate of 2-pentylcyclopentan-1-one (7) obtained after the reaction for 2 hours.

**Table 5**

| | Ex. 10 | Com.Ex. 3 | Com.Ex. 4 |
|---|---|---|---|
| Platinum group metal catalyst (mass% with respect to Compound (5)) | 5% Pd/C (1.1 mass%) | 5% Pd/C (3.3 mass%) | 5% Pd/C (1.1 mass%) |
| Nitrogen : Hydrogen | 1:1.5 | | |
| Simple substance of Group 16 element (mol% with respect to metal content of platinum group metal catalyst) | Sulfur powder (13 mol%) | - | - - |
| Reaction temperature | 140°C | | |
| Reaction time (hours) | 2 | 4 | 2 |
| Yield (mol%) of Compound (3) | 98 | 93 | 50 |
| Formation rate (mol%) of Compound (7) | 2 | 6 | 3 |

It is seen from comparison between Example 10 and Comparative Example 3 that the amount of metal catalyst can be reduced and the reaction time can be shortened.

It is seen from comparison between Example 10 and Comparative Example 4 having the same amount of metal catalyst that the yield of the isomerization reaction can be improved.

### Example 11

2-(1-Hydroxypentyl)-cyclopentan-1-one (4) (300 g, purity 87%) obtained in Reference Example 1, phosphoric acid-supported activated carbon (powder, 3.3 g, 1.3 mass% with respect to the amount of the compound (4)), 5% Pd/C (powder, water content 60.9%, 3.84 g, 0.6 mass% with respect to the amount of the compound (4)), and a sulfur powder (manufactured by Fujifilm Wako Pure Chemical Corporation, powder, 2.9 mg, 13 mol% with respect to the metal content of the platinum group metal catalyst) were added to a 500-mL four-necked separable flask (made of glass) equipped with a connecting tube, and the mixture was stirred at 400 rpm using a 6-bladed disk turbine blade (blade diameter 3 cm) and heated and mixed to 101 kPa, 140°C in an atmosphere of nitrogen : hydrogen (volume ratio) = 1 : 0.43. During the reaction, the distillate was continuously distilled into a distillate receiver connected to the above-mentioned flask. After the reaction for 15 hours, 38.4 g of distillate was obtained and 254.6 g of reaction end product was obtained. The obtained reaction end product was quantified using GC. Table 6 shows the yield of 2-pentyl-2-cyclopenten-1-one (3) obtained and the formation rate of 2-pentylcyclopentan-1-one (7). The reaction end product was filtered using a pressure filter to recover the catalyst (5% Pd/C).

### Example 12

The procedure was the same as that for Example 11, except that the catalyst recovered in Example 11 was used instead of adding phosphoric acid-supported activated carbon, 5% Pd/C, and a sulfur powder in Example 11.

Table 6 shows the yield of 2-pentyl-2-cyclopenten-1-one (3) obtained and the formation rate of 2-pentylcyclopentan-1-one (7).

### Example 13

The procedure was the same as that for Example 12, except that the catalyst recovered in Example 12 was used instead of the catalyst recovered in Example 11. Table 6 shows the yield of 2-pentyl-2-cyclopenten-1-one (3) obtained and the formation rate of 2-pentylcyclopentan-1-one (7).

### Example 14

The procedure was the same as that for Example 13, except that the catalyst recovered in Example 13 was used instead of the catalyst recovered in Example 12. Table 6 shows the yield of 2-pentyl-2-cyclopenten-1-one (3) obtained and the formation rate of 2-pentylcyclopentan-1-one (7).

It is seen from Table 6 that the use of a sulfur powder as a simple substance of a Group 16 element, as a solid catalyst, does not reduce the yield even after three reuses.

**Table 6**

| | Ex. 11 | Ex. 12 | Ex. 13 | Ex. 14 |
|---|---|---|---|---|
| Solid acid (mass% with respect to Compound (4)) | Phosphoric acid-supported activated carbon (1.3 mass%) | Catalyst recovered in Ex. 11 | Catalyst recovered in Ex. 12 | Catalyst recovered in Ex. 13 |
| Platinum group metal catalyst (mass% with respect to Compound (4)) | 5% Pd/C (0.6 mass%) | Catalyst recovered in Ex. 11 | Catalyst recovered in Ex. 12 | Catalyst recovered in Ex. 13 |
| Nitrogen : Hydrogen | 1.0 : 0.43 | | | |
| Simple substance of Group 16 element (mol% with respect to metal content of platinum group metal catalyst) | Sulfur powder (13 mol%) | Catalyst recovered in Ex. 11 | Catalyst recovered in Ex. 12 | Catalyst recovered in Ex. 13 |
| Reaction temperature | 140°C | | | |
| Reaction time (hours) | 15 | 15 | 18 | 15 |
| Yield (%) of Compound (3) | 87% | 87% | 88% | 87% |
| Formation rate (%) of Compound (7) | 5% | 4% | 4% | 4% |

## Claims

1. A method for producing a compound represented by General Formula (I) below, comprising a step of isomerizing a compound represented by General Formula (II) below in the presence of molecular hydrogen and/or a hydrogen source, a platinum group metal catalyst, and a simple substance of a Group 16 element (excluding oxygen) or a compound thereof. 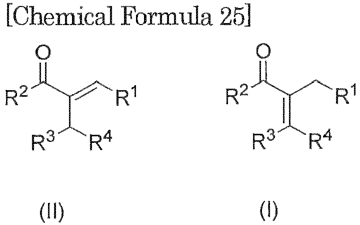
wherein R¹, R², and R⁴ simultaneously or independently represent a hydrogen atom, a linear or branched alkyl group having 1 to 8 carbon atoms, or a linear or branched alkenyl group having 2 to 8 carbon atoms, and
R³ represents a linear or branched alkyl group having 1 to 8 carbon atoms or a linear or branched alkenyl group having 2 to 8 carbon atoms, or
R¹ and R⁴ simultaneously or independently represent a hydrogen atom, a linear or branched alkyl group having 1 to 8 carbon atoms, or a linear or branched alkenyl group having 2 to 8 carbon atoms, and
R² and R³, taken together, represent an alkanediyl group having 2 to 9 carbon atoms or represent an alkanediyl group having 2 to 9 carbon atoms substituted with one or two alkyl groups having 1 to 5 carbon atoms.

2. The producing method according to claim 1, wherein R² and R³, taken together, represent an alkanediyl group having 2 to 9 carbon atoms.

3. The producing method according to claim 1 or 2, wherein a metal of the platinum group metal catalyst is palladium or platinum.

4. The producing method according to any one of claims 1 to 3, wherein a metal of the platinum group metal catalyst is supported on carbon.

5. The producing method according to any one of claims 1 to 4, wherein the simple substance of the Group 16 element (excluding oxygen) or the compound thereof includes one or more selected from the group consisting of sulfur and a sulfur compound, selenium and a selenium compound, and tellurium and a tellurium compound.

6. The producing method according to claim 5, wherein the sulfur compound is a compound having a sulfide group, a thiophene ring, or a thiol group.

7. The producing method according to any one of claims 1 to 4, wherein the simple substance of the Group 16 element (excluding oxygen) or the compound thereof includes sulfur.

8. The producing method according to any one of claims 1 to 7,
wherein the molecular hydrogen is mixed with inert gas, and
the inert gas is one or more selected from the group consisting of nitrogen, argon, and helium.

9. The producing method according to claim 8, wherein a volume ratio of the molecular hydrogen to the inert gas (molecular hydrogen / inert gas) is 10/1 to 1/10.

10. The producing method according to any one of claims 1 to 9, wherein the amount of the simple substance of the Group 16 element (excluding oxygen) or the compound thereof used is 0.1 mol% or more and 100 mol% or less with respect to a metal content of the platinum group metal catalyst.

11. The producing method according to any one of claims 1 to 10, further comprising a step of dehydrating the compound represented by General Formula (III) above in the presence of an acid, thereby obtaining the compound represented by General Formula (II) above: 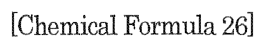 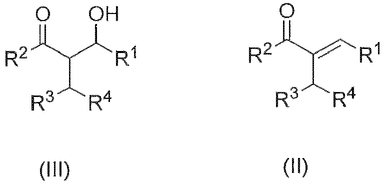
wherein R¹, R², and R⁴ simultaneously or independently represent a hydrogen atom, a linear or branched alkyl group having 1 to 8 carbon atoms, or a linear or branched alkenyl group having 2 to 8 carbon atoms, and
R³ represents a linear or branched alkyl group having 1 to 8 carbon atoms or a linear or branched alkenyl group having 2 to 8 carbon atoms, or
R¹ and R⁴ simultaneously or independently represent a hydrogen atom, a linear or branched alkyl group having 1 to 8 carbon atoms, or a linear or branched alkenyl group having 2 to 8 carbon atoms, and
R² and R³, taken together, represent an alkanediyl group having 2 to 9 carbon atoms or represent an alkanediyl group having 2 to 9 carbon atoms substituted with one or two alkyl groups having 1 to 5 carbon atoms.

12. The producing method according to claim 11, wherein the acid includes a solid acid.

13. The producing method according to any one of claims 1 to 12, wherein R¹ is an n-butyl group, R² and R³, taken together, are an alkanediyl group having 2 carbon atoms, and R⁴ is a hydrogen atom.

14. The producing method according to any one of claims 1 to 13, wherein the compound represented by General Formula (III) above is 2-(1-hydroxypentyl)-cyclopentan-1-one.

15. A method for producing a compound of Formula (VII), comprising Steps 1 and 2 below:
Step 1: Step of dehydrating and isomerizing a compound represented by General Formula (IV) below in the presence of molecular hydrogen and/or a hydrogen source, an acid, a platinum group metal catalyst, and a simple substance of a Group 16 element (excluding oxygen) or a compound thereof, thereby obtaining a compound represented by General Formula (V) below
Step 2: Step of reacting the compound represented by General Formula (V) obtained in Step 1 with a malonic acid diester represented by General Formula (VI) below and then with water, thereby obtaining a compound represented by General Formula (VII) below 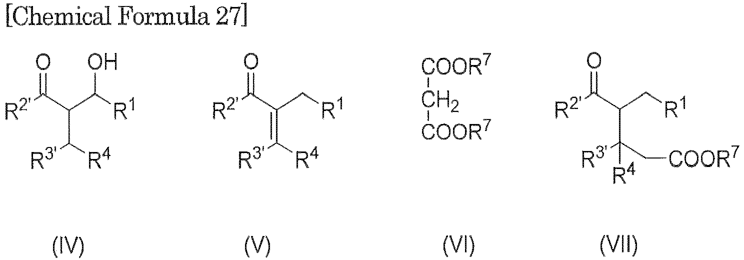
wherein R¹ and R⁴ simultaneously or independently represent a hydrogen atom, a linear or branched alkyl group having 1 to 8 carbon atoms, or a linear or branched alkenyl group having 2 to 8 carbon atoms,
R²' and R³', taken together, represent an alkanediyl group having 2 to 9 carbon atoms or represent an alkanediyl group having 2 to 9 carbon atoms substituted with one or two alkyl groups having 1 to 5 carbon atoms, and
R⁷ represents an alkyl group having 1 to 3 carbon atoms, and two R⁷s are optionally the same or different.

16. The producing method according to claim 15, wherein R¹ is an n-butyl group, R²' and R³', taken together, are an alkanediyl group having 2 carbon atoms, R⁴ is a hydrogen atom, and R⁷ is a methyl group.
